(19) Europäisches Patentamt / European Patent Office / Office européen des brevets

(11) **EP 3 678 532 B1**

(12) **FASCICULE DE BREVET EUROPEEN**

(45) Date de publication et mention de la délivrance du brevet:
**09.10.2024 Bulletin 2024/41**

(21) Numéro de dépôt: **18778371.7**

(22) Date de dépôt: **06.09.2018**

(51) Classification Internationale des Brevets (IPC):
**A61B 5/00** *(2006.01)*    **A61B 5/16** *(2006.01)*

(52) Classification Coopérative des Brevets (CPC):
**A61B 5/168; A61B 5/369; A61B 5/7246; A61B 5/7267; A61B 5/742; G16H 50/20**

(86) Numéro de dépôt international:
**PCT/EP2018/073961**

(87) Numéro de publication internationale:
**WO 2019/048525 (14.03.2019 Gazette 2019/11)**

(54) **DECODAGE DE L'ATTENTION VISUELLE D'UN INDIVIDU A PARTIR DE SIGNAUX ELECTROENCEPHALOGRAPHIQUES**

DECODIERUNG DER VISUELLEN AUFMERKSAMKEIT EINES INDIVIDUUMS AUS ELEKTROENZEPHALOGRAPHISCHEN SIGNALEN

DECODING THE VISUAL ATTENTION OF AN INDIVIDUAL FROM ELECTROENCEPHALOGRAPHIC SIGNALS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.09.2017 FR 1758305**

(43) Date de publication de la demande:
**15.07.2020 Bulletin 2020/29**

(73) Titulaire: **Nextmind SAS**
**75009 Paris (FR)**

(72) Inventeurs:
• **KOUIDER, Sid**
**75001 Paris (FR)**
• **LEONETTI, Jean-Maurice**
**91240 Saint Michel Sur Orge (FR)**
• **BARASCUD, Nicolas**
**75014 Paris (FR)**
• **ZERAFA, Robin**
**94110 Arceuil (FR)**

(74) Mandataire: **Kramer, Dani et al**
**Mathys & Squire**
**The Shard**
**32 London Bridge Street**
**London SE1 9SG (GB)**

(56) Documents cités:
**US-B2- 8 391 966**

• **GUANGYU BIN ET AL: "An online multi-channel SSVEP-based brain-computer interface using a canonical correlation analysis method; An online multi-channel SSVEP-based BCI using the CCA method", JOURNAL OF NEURAL ENGINEERING, INSTITUTE OF PHYSICS PUBLISHING, BRISTOL, GB, vol. 6, no. 4, 1 August 2009 (2009-08-01), pages 46002, XP020162289, ISSN: 1741-2552**
• **YIN ERWEI ET AL: "A Dynamically Optimized SSVEP Brain-Computer Interface (BCI) Spe", IEEE TRANSACTIONS ON BIOMEDICAL ENGINEERING, IEEE SERVICE CENTER, PISCATAWAY, NJ, USA, vol. 62, no. 6, 1 June 2015 (2015-06-01), pages 1447 - 1456, XP011581726, ISSN: 0018-9294, [retrieved on 20150518], DOI: 10.1109/TBME.2014.2320948**

**Description**

DOMAINE TECHNIQUE

La présente description concerne un procédé et système de détermination de la focalisation de l'attention visuelle d'un individu à partir de signaux électroencéphalographiques.

ETAT DE L'ART

On peut constater l'émergence de systèmes portatifs variés dédiés à l'enregistrement et l'exploitation de signaux électroencéphalographiques (signaux EEG) dans de multiples applications. En particulier, la miniaturisation des systèmes d'enregistrement de signaux EEG et les importants développements des techniques d'analyse liées au décodage temps-réel des signaux EEG permettent désormais d'envisager de nouvelles applications à la fois rapides et fiables dans leur utilisation.

Certaines techniques de décodage reposent sur une extraction des signaux EEG de descripteurs électro-physiologiques permettant de prédire en temps réel les relations entre l'activité cérébrale et les stimuli visuels dans l'environnement. La difficulté consiste ici à identifier, dans le signal EEG et en temps-réel, les descripteurs propres à un stimulus visuel spécifique auquel un individu porte son attention parmi une multitude d'autres contenus visuels. Un tel décodage doit être robuste, c'est-à-dire doit permettre, de manière rapide et précise, de déterminer vers quel contenu spécifique est portée l'attention visuelle de l'individu afin de pouvoir déclencher une commande correspondant au stimulus visuel focalisé par l'individu.

Le document de brevet US8391966B2 décrit une technique d'analyse de signaux EEG produits par un individu observant des stimuli, chaque stimulus étant constitué par une source lumineuse clignotante à une fréquence donnée. Différentes sortes de descripteurs sont générés pour permettre une classification des signaux EEG en classes correspondant aux différents stimuli en vue d'une identification du stimulus visuel observé à un instant donné.

Les signaux EEG sont par exemple découpés en segments successifs et des coefficients de corrélation entre des pairs de segments d'un même signal sont calculés pour produire un premier type de descripteur. Un coefficient de corrélation moyen est calculé puis comparé à un seuil pour déterminer si l'utilisateur observe ou non le stimulus. Par ailleurs, la corrélation entre un signal EEG et le stimulus peut être analysée pour générer un deuxième type de descripteur : le degré de corrélation avec un stimulus sera plus élevé si l'individu observe effectivement ce stimulus. Les coefficients d'un modèle autorégressif peuvent être calculés à partir d'un signal EEG moyen, les coefficients du modèle constituant un troisième type de descripteur.

Cette technique suppose une classification préalable des EEG au moyen de plusieurs types de descripteurs en association avec une méthode de discrimination par seuillage, recherche des plus proches voisins, réseaux de neurones, etc. La technique est donc dépendante de la pertinence des descripteurs utilisés et de la méthode de discrimination choisie.

De surcroît, la technique est limitée à des stimuli sous forme de lumières clignotantes, ce qui limite fortement le champ d'application.

D'autres méthodes sont connues. Par exemple, le document intitulé « An online multi-channel SSVEP-based brain computer interface using canonical corrélation analysis method », par Guangyu Bin et al, (Journal of Neural Engineering, IOP Publishing, 2009, utilise une méthode d'analyse de corrélation canonique (CCA) modifiée.

Il apparaît ainsi un besoin de disposer d'une technique qui permette un décodage fiable et en temps réel des signaux EEG pour réaliser une interface cerveau-machine entre un usager et une application logicielle, comprenant par exemple du texte, des images et/ou des menus.

RESUME

La présente description a pour objet, selon un premier aspect, un procédé de détermination de la focalisation de l'attention visuelle d'un individu à partir de signaux électroencéphalographiques. Le procédé comprend une génération d'un ensemble d'au moins un stimulus visuel à afficher à partir d'au moins un objet graphique, d'au moins une transformation élémentaire et d'un ensemble d'au moins un signal de modulation, un stimulus visuel étant un objet graphique animé obtenu par application à un objet graphique d'une séquence temporelle de transformations élémentaires paramétrée temporellement par un signal de modulation correspondant; une reconstruction d'un signal de modulation à partir d'une pluralité de signaux électroencéphalographiques produits par l'individu pour obtenir un signal de modulation reconstruit ; un calcul d'un degré de dépendance statistique entre le signal de modulation reconstruit et chaque signal de modulation de l'ensemble d'au moins un signal de modulation; une identification d'au moins stimulus visuel correspondant à un signal de modulation pour lequel le degré de dépendance statistique est supérieur à un premier seuil.

Le procédé selon le premier aspect consiste en une méthode de décodage hybride qui combine une approche par reconstruction de stimulus à partir des signaux électroencéphalographiques (EEG) avec une méthode d'excitation au moyen d'un ou plusieurs stimuli ayant les caractéristiques temporelles données, aptes à être reproduites au niveau du cerveau d'un individu observant ce ou ces stimuli, ces caractéristiques temporelles se retrouvant dans les signaux EEG et étant donc identifiables directement dans les signaux EEG. Une telle combinaison permet à la fois d'augmenter la sensibilité (c'est-à-dire le rapport signal sur bruit) du signal EEG aux stimuli générés et de disposer d'une méthode robuste d'analyse applicable en temps réel. Le procédé est applicable à un ou plusieurs stimuli visuels.

En outre, l'utilisation de transformations élémentaires (par exemple, une variation d'intensité lumineuse, une variation de contraste, une transformation colorimétrique, une déformation géométrique, une rotation, une oscillation, un déplacement selon une trajectoire, etc.) permet de disposer d'une gamme étendue de stimuli visuels sous forme d'objet graphiques qui ouvre la porte à de nombreuses applications nécessitant de faire la distinction entre de nombreux objet graphiques présentés simultanément à un utilisateur.

La technique est ainsi adaptable par exemple à un affichage de clavier de caractères alphanumériques en vue de l'identification du caractère alphanumérique sur lequel l'attention d'un utilisateur est focalisée ou plus généralement à un affichage d'une pluralité de logos, menus, éléments graphiques, etc. La modulation apportée par le signal de modulation n'affecte pas le confort de vision pour autant que les composantes fréquentielles de ce signal de modulation soient inférieures à 25Hz environ. Un signal de modulation présente par exemple un motif périodique, se répétant avec fréquence comprise entre 2 et 20 Hz, ce signal de modulation étant échantillonné à une fréquence d'échantillonnage correspondant à la fréquence de rafraîchissement (en général, supérieure à 60Hz) de l'écran sur lequel les stimuli sont affichés.

La reconstruction et recherche de dépendance statistique peuvent être effectuées en temps réel, ce qui permet d'identifier en temps réel l'objet graphique sur lequel l'individu porte son attention. En particulier, aucune classification préalable des signaux EEG en classes correspondant respectivement aux différents stimuli n'est requise pour la reconstruction du signal de modulation.

Selon un ou plusieurs modes de réalisation du procédé selon le premier aspect, l'ensemble d'au moins un stimulus visuel comprend une pluralité de stimuli visuels et l'ensemble d'au moins signal de modulation comprend une pluralité de signaux de modulation et le procédé comprend en outre une recherche , parmi la pluralité de signaux de modulation, d'un signal de modulation pour lequel le degré de dépendance statistique avec le signal de modulation reconstruit est maximal ; et une identification du stimulus visuel correspondant au signal de modulation pour lequel le degré de dépendance statistique est maximal ; les signaux de modulations étant composés de sorte qu'un degré de dépendance statistique global, déterminé dans le domaine temporel et/ou fréquentiel, pour toutes les paires de signaux de modulation correspondant à deux stimuli visuels distincts, soit inférieur à un deuxième seuil.

Selon un ou plusieurs modes de réalisation du procédé selon le premier aspect, la reconstruction est effectuée par application d'un modèle de reconstruction à la pluralité de signaux électroencéphalographiques.

Le modèle de reconstruction établit la relation mathématique existant entre un signal de modulation utilisé pour la génération d'un stimulus visuel et la réponse électroencéphalographique de l'individu focalisant son attention sur ce stimulus visuel. Le modèle de reconstruction sert ainsi à extraire des signaux EEG les informations pertinentes par rapport à un type de stimulus. Cette relation est dépendante principalement de la position sur le crâne d'un individu (dénommé également, utilisateur) des électrodes de l'équipement d'acquisition des signaux EEG. Les caractéristiques visuelles des stimuli (type, taille, position, couleur, etc.) ont en effet un impact réduit sur cette relation.

Le modèle de reconstruction est susceptible d'utiliser divers modèles mathématiques, linéaires ou non, permettant de combiner les signaux EEG pour reconstruire un signal de modulation. Du fait que l'on reconstruit un signal de modulation, et non pas le stimulus visuel en tant que tel (c'est-à-dire, l'objet graphique animé qui est affiché sur un écran), la reconstruction est possible simplement et avec précision, par exemple par simple combinaison linéaire des signaux EEG, et ce sans restriction quant à la nature ou au contenu sémantique de l'objet graphique animé, utilisable comme stimulus visuel.

La recherche de dépendance statistique entre le signal de modulation reconstruit et le ou les différents signaux de modulation est également facilitée. En outre, le ou les stimuli visuels sont affichables sur tous les écrans couramment disponibles, tels que : écran d'ordinateur, écran de tablette, écran de terminal de téléphonie, etc. Il n'est donc pas nécessaire de disposer de système de production de stimuli dédié. Ainsi l'utilisation de signaux de modulation pour la génération de stimuli visuels permet non seulement une reconstruction et recherche de dépendance statistique aisée, mais également rend la méthode souple et adaptable à tout type de stimuli visuels sous forme d'objet graphique animé.

Selon un ou plusieurs modes de réalisation du procédé selon le premier aspect, le modèle de reconstruction comprend une pluralité de paramètres de combinaison de signaux électroencéphalographiques et le procédé comprend une détermination de valeurs des paramètres de la pluralité de paramètres de combinaison de signaux électroencéphalographiques lors d'une phase initiale d'apprentissage.

Selon un ou plusieurs modes de réalisation du procédé selon le premier aspect, le procédé comprend en outre, lors d'une phase initiale d'apprentissage appliquée à un sous-ensemble d'au moins un stimulus visuel parmi la pluralité de stimuli visuels, une obtention, pour chaque stimulus visuel dudit sous-ensemble d'au moins un stimulus visuel, de signaux électroencéphalographiques de tests produits par l'individu focalisant son attention sur le stimulus visuel considéré; et une détermination de valeurs optimales de la pluralité de paramètres de combinaison de signaux électroencéphalographiques pour lesquelles l'application du modèle de reconstruction à la pluralité de signaux électroencéphalographiques de tests enregistrés pour un stimulus visuel permet de générer un signal de modulation reconstruit approchant au mieux le signal de modulation correspondant au stimulus visuel considéré.

Ainsi, en déterminant les paramètres de combinaison des signaux EEG pour un dispositif d'acquisition donné, on peut de manière fiable générer un signal de modulation reconstruit et le comparer à ceux utilisés pour la génération des stimuli visuels. Cette relation étant stable dans le temps, et peu dépendante des stimuli visuels et des objets graphiques, ces paramètres de combinaison des signaux EEG sont réutilisables pour tous les stimuli visuels ultérieurs susceptibles d'être présentés à un individu, mêmes si ces stimuli sont différents de ceux utilisés pour en phase d'apprentissage pour la détermination du modèle de reconstruction.

Le modèle de reconstruction permet enfin de gérer une éventuelle variabilité d'un individu à un autre en ce que les paramètres du modèle de reconstruction peuvent être ajustés pour chaque individu.

Selon un ou plusieurs modes de réalisation, une transformation élémentaire est une transformation de l'ensemble de transformations constitué par une variation d'intensité lumineuse, une variation de contraste, une transformation colorimétrique, une déformation géométrique, une rotation, une oscillation, un déplacement selon une trajectoire, un changement de forme et un changement d'objet graphique ou une combinaison de transformations choisies dans ledit ensemble de transformations.

La présente description a pour objet, selon un deuxième aspect, un programme informatique comprenant des instructions de code pour l'exécution des étapes d'un procédé selon le premier aspect, lorsque ce programme informatique est exécuté par un processeur de données.

La présente description a pour objet, selon un troisième aspect, un système informatique comprenant au moins une mémoire de stockage d'instructions de code d'un programme d'ordinateur configuré pour l'exécution d'un procédé selon le premier aspect et au moins un processeur de données configuré pour exécuter un tel programme d'ordinateur.

La présente description a pour objet, selon un quatrième aspect, un système de détermination de la focalisation de l'attention visuelle d'un individu à partir de signaux électroencéphalographiques, le système comprenant des moyens de mise en oeuvre du procédé selon le premier aspect, selon l'un quelconque des modes de réalisation décrit.

Le système comprend notamment un dispositif de génération de signaux d'affichage configuré pour générer un ensemble d'au moins un stimulus visuel à afficher à partir d'au moins un objet graphique, d'au moins une transformation élémentaire et d'un ensemble d'au moins un signal de modulation, un stimulus visuel étant un objet graphique animé obtenu par application à un objet graphique d'une séquence temporelle de transformations élémentaires paramétrée temporellement par un signal de modulation correspondant; un dispositif de traitement de signaux configuré pour obtenir une pluralité de signaux électroencéphalographiques produits par l'individu; obtenir un signal de modulation reconstruit par reconstruction d'un signal de modulation à partir de la pluralité de signaux électroencéphalographiques; calculer un degré de dépendance statistique entre le signal de modulation reconstruit et chaque signal de modulation dudit ensemble d'au moins un signal de modulation; et identifier au moins un stimulus visuel correspondant à un signal de modulation pour lequel le degré de dépendance statistique est supérieur à un premier seuil.

Selon un ou plusieurs modes de réalisation du système selon le quatrième aspect, l'ensemble d'au moins un stimulus visuel comprend une pluralité de stimuli visuels et l'ensemble d'au moins signal de modulation comprend une pluralité de signaux de modulation et le système de traitement de signaux est en outre configuré pour rechercher, parmi la pluralité de signaux de modulation, le signal de modulation pour lequel le degré de dépendance statistique avec le signal de modulation reconstruit est maximal ; identifier le stimulus visuel correspondant au signal de modulation pour lequel le degré de dépendance statistique est maximal ; les signaux de modulations étant composés de sorte qu'un degré de dépendance statistique global, déterminé dans le domaine temporel et/ou fréquentiel, pour toutes les paires de signaux de modulation correspondant à deux stimuli visuels distincts, soit inférieur à un deuxième seuil.

BREVE DESCRIPTION DES FIGS.

D'autres avantages et caractéristiques de la technique présentée ci-dessus apparaîtront à la lecture de la description détaillée ci-dessous, faite par référence aux FIGS. dans lesquelles :

- la FIG. 1A représente de manière schématique un système de détermination de la focalisation de l'attention visuelle d'un individu à partir de signaux EEG selon un exemple de réalisation;
- la FIG. 1B représente de manière schématique un dispositif informatique selon un exemple de réalisation;
- la FIG. 2A représente de manière schématique les données et signaux exploités dans un système et procédé de détermination de la focalisation de l'attention visuelle selon un exemple de réalisation;
- les FIG. 2B-2E représente chacune de manière schématique des exemples de signaux de modulations utilisables dans un procédé ou système de détermination de la focalisation de l'attention visuelle;

- la FIG. 3 représente de manière schématique des aspects d'un procédé et système de détermination de la focalisation de l'attention visuelle;
- la FIG. 4A est un organigramme d'un procédé de génération d'un modèle de reconstruction de signaux EEG selon un exemple de réalisation;
- la FIG. 4B est un organigramme d'un procédé de détermination de la focalisation de l'attention visuelle d'un individu selon un exemple de réalisation ;
- la FIG. 5 illustre un exemple d'objet graphique animé
- la FIG. 6 montre des exemples de stimuli visuels ;
- la FIG. 7 illustre un exemple d'application d'un système et procédé de détermination de la focalisation de l'attention visuelle.

Dans les différents modes de réalisation qui vont être décrits par référence aux FIGS., des éléments semblables ou identiques portent les mêmes références.

## DESCRIPTION DETAILLEE

La présente description, est faite par référence à des fonctions, unités fonctionnelles, entités, schémas blocs et organigrammes qui décrivent différents modes de réalisation de procédés, systèmes et programmes. Chaque fonction, unité fonctionnelle, entité, étape d'un organigramme peut être mis en oeuvre par logiciel, hardware, firmware, microcode ou toute combinaison appropriée de ces technologies. Lorsqu'un logiciel est utilisé, les fonctions, unités fonctionnelles, entités ou étapes peuvent être mises en oeuvre par des instructions de programme d'ordinateur ou du code logiciel. Ces instructions peuvent être stockées ou transmises vers un support de stockage lisible par un ordinateur et/ou être exécutées par un ordinateur afin de mettre en oeuvre ces fonctions, unités fonctionnelles, entités ou étapes.

Les différents modes de réalisation et aspects décrits ci-dessous peuvent être combinés ou simplifiés de multiples manières. En particulier, les étapes des différents procédés peuvent être répétées pour chaque ensemble d'objets graphiques concerné et/ou chaque utilisateur concerné, les étapes peuvent être interverties, exécutées en parallèle, exécutées par différentes entités informatiques. Seuls certains modes de réalisation d'exemples sont décrits en détail pour assurer la clarté de l'exposé mais ces exemples ne visent pas à limiter la portée générale des principes ressortant de cette description considérée dans son ensemble.

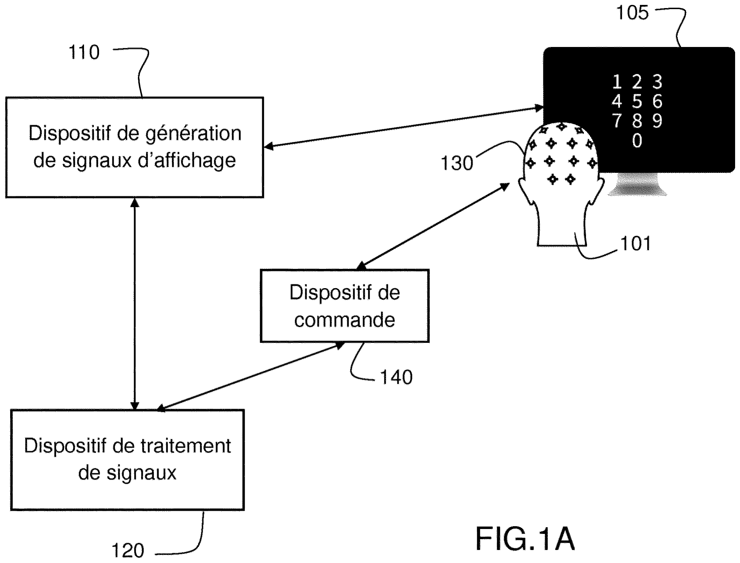

La FIG. 1A représente de manière schématique un exemple de réalisation d'un système 100 de détermination de la focalisation de l'attention visuelle d'un individu (dénommé également ci-après, utilisateur) 101 à partir de signaux électroencéphalographiques.

Dans un ou plusieurs modes de réalisation, le système 100 comprend un écran d'affichage 105 configuré pour afficher des objets graphiques animés, un dispositif 110 de génération de signaux d'affichage, un dispositif 120 de traitement de signaux, un équipement 130 d'acquisition de signaux EEG et un dispositif 140 de commande de l'équipement 130.

Dans un ou plusieurs modes de réalisation, le dispositif 110 de génération de signaux d'affichage est configuré pour générer des signaux d'affichage à afficher par l'écran d'affichage 105. Ces signaux d'affichage encodent une pluralité de stimuli visuels destinés à être présentés à l'utilisateur 101 au moyen de l'écran d'affichage 105.

Dans un ou plusieurs modes de réalisation, l'équipement 130 est configuré pour l'acquisition de signaux EEG. Cet équipement est par exemple réalisé sous forme de casque, muni d'électrodes destinées à entrer en contact avec le crâne de l'utilisateur 101. Un tel casque est par exemple un casque fabriqué par la société Biosemi ®, muni de 64 électrodes. D'autres types équipements sont utilisables : par exemple les dispositifs EEG Geodesic™ de la société Electrical Geodesics Inc. (EGI) ®, ou ceux de la société Compumedics NeuroScan ® comptant habituellement entre 16 et 256 électrodes. Dans la suite de la description, on suppose à titre d'exemple que l'équipement 130 est réalisé sous forme de casque.

Dans un ou plusieurs modes de réalisation, le dispositif 120 de traitement de signaux est configuré pour traiter les signaux EEG acquis au moyen du casque 130 d'acquisition de signaux EEG.

Dans un ou plusieurs modes de réalisation, le dispositif 140 de commande de l'équipement 130 est un dispositif servant d'interface entre le casque 130 et/ou le dispositif 120 de traitement de signaux. Le dispositif 140 de commande est configuré pour commander l'acquisition de signaux EEG et pour obtenir les signaux EEG acquis par le casque 130. En particulier, le dispositif 140 de commande du casque 130 est configuré pour envoyer une commande de déclenchement de l'acquisition de signaux EEG.

Tout ou partie des fonctions décrites ici pour le dispositif 110 de génération de signaux d'affichage, le dispositif 120 de traitement de signaux et le dispositif 140 de commande peuvent être réalisées par logiciel et/ou hardware et mise en oeuvre dans au moins un dispositif informatique comprenant un processeur de données et au moins une mémoire de stockage de données.

Dans un ou plusieurs modes de réalisation, chaque dispositif 110, 120, 140 et chacune des étapes des procédés décrits sont mis en oeuvre par un ou plusieurs dispositifs informatiques physiquement distincts. Inversement, les différents dispositifs 110, 120, 140 peuvent être intégrés dans un seul et même dispositif informatique. De même les étapes des procédés décrits ici peuvent être mises en oeuvre par un seul et même dispositif informatique. L'équipement 130 d'acquisition de signaux EEG peut également comprendre un dispositif informatique et être configuré (processeur de données, mémoire, etc) pour mettre en oeuvre tout ou partie des étapes des procédés décrits dans ce document.

Chaque dispositif informatique présente globalement l'architecture d'un ordinateur, incluant des constituants d'une telle architecture : mémoire(s) de données, processeur(s), bus de communication, interface(s) utilisateur, interface(s) matérielle(s) pour la connexion de ce dispositif informatique à un réseau ou un autre équipement, etc.

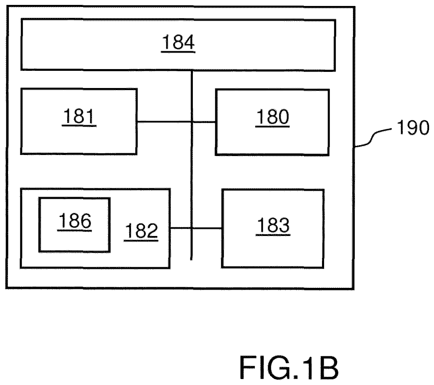

Un exemple de réalisation d'une telle architecture est illustré à la figure 1B. Cette architecture comprend une unité de traitement 180 incluant au moins un processeur de données, au moins une mémoire 181, un ou plusieurs supports de stockage de données 182, et des interfaces hardware 183 telles que des interfaces réseaux, des interfaces pour la connexion de périphériques, au moins une interface utilisateur 184 incluant un ou plusieurs dispositifs d'entrée / sortie tels que souris, clavier, affichage, etc. Le support de stockage de données 182 comprend des instructions de code d'un programme d'ordinateur 186. Un tel support de stockage 182 peut être un dispositif de stockage optique tels que disque compact (CD, CD-R ou CD-RW), DVD (DVD-Rom ou DVD-RW) ou Blu-ray, un support magnétique tel que disque dur, disque à mémoire flash, bande magnétique ou disquette, un support de stockage amovible de type clef USB, une carte à mémoire SD ou micro SD, etc. La mémoire 181 peut être une mémoire vive (RAM), une mémoire morte (ROM), une mémoire cache, une mémoire rémanente, une mémoire de sauvegarde (par exemple des mémoires programmables ou flash), des mémoires mortes ou n'importe quelle combinaison de ces types de mémoire. L'unité de traitement 180 peut être n'importe quel microprocesseur, circuit intégré, ou unité centrale comportant au moins un processeur de traitement à base de matériel informatique.

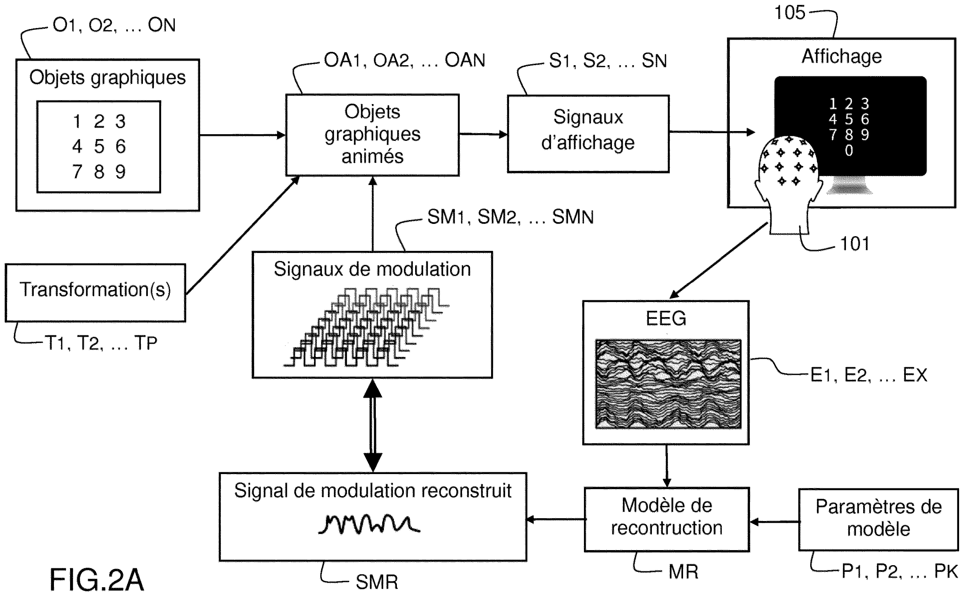

La FIG. 2A représente de manière schématique les données et signaux exploités dans un système et procédé de détermination de la focalisation de l'attention visuelle. Les notations correspondantes sont introduites.

Dans un ou plusieurs modes de réalisation, une pluralité d'objets graphiques O1, O2, ..., ON destinés à être présentés à un utilisateur 101 est utilisée. Chacun de ces objets graphiques peut être un caractère alphanumérique (chiffre ou lettre ou autre caractère), un logo, une image, un texte, un élément de menu d'interface utilisateur, un bouton d'interface utilisateur, un avatar, un objet 3D, etc. Chacun de ces objets graphiques peut être codé par une image bitmap ou vectorielle.

Dans un ou plusieurs modes de réalisation, une ou plusieurs transformations élémentaires T1, T2, ..., TP sont définies pour être appliquées aux objets graphiques O1, O2, ..., ON. Une transformation élémentaire peut être : une variation d'intensité lumineuse, une variation de contraste, une transformation colorimétrique, une déformation géométrique, une rotation, une oscillation, un déplacement selon une trajectoire plane ou tridimensionnelle, un changement de forme, voire un changement d'objet graphique etc. Un changement d'objet graphique peut par exemple correspondre à une transformation qui remplace un objet graphique par un autre objet graphique de la même catégorie, par exemple, le remplacement d'une lettre (par ex. A) par une autre lettre (par ex. B), d'un chiffre par un autre chiffre, un logo par un autre logo, etc. Une transformation élémentaire peut être également une combinaison de plusieurs des transformations élémentaires précitées.

Chacune de ces transformations élémentaires est paramétrable par au moins un paramètre d'application.

Dans un ou plusieurs modes de réalisation, un paramètre d'application définit un degré de transformation de la transformation élémentaire sur une échelle prédéterminée. Une échelle de 0 à 100 ou de -100 à + 100 peut par exemple être utilisée.

Par exemple, lorsque la transformation élémentaire est une variation d'intensité lumineuse, cette variation d'intensité peut être appliquée avec un degré de transformation variable entre 0 et 100, un degré de transformation égal à 0 signifiant que l'image codant l'objet graphique n'est pas modifiée, un degré de transformation égal à 100 indiquant que l'image devient complètement blanche ou, au contraire, noire. En faisant varier le degré de transformation entre 0 et 100, on obtient un effet de clignotement de l'image.

Selon un autre exemple, lorsque la transformation élémentaire est une variation de contraste, cette variation de contraste peut être appliquée avec un degré de transformation variable entre 0 et 100, un degré de transformation égal à 0 signifiant que l'image codant l'objet graphique n'est pas modifiée, un degré de transformation égal à 100 indiquant que le contraste de l'image devient maximal (l'image devient une image noire et blanche, si elle est codée en niveau de gris).

De même, pour une déformation géométrique de type morphing, un degré de transformation peut correspondre au degré de morphing. Pour une rotation, un degré de transformation peut correspondre à un angle de rotation. Pour une oscillation, un degré de transformation peut correspondre à une vitesse et/ou amplitude d'oscillation. Pour un déplacement sur une trajectoire, un degré de transformation peut correspondre à une distance parcourue et/ou vitesse de déplacement sur la trajectoire. Pour un changement de forme (respectivement de catégorie d'objet), un degré de transformation peut correspondre à une vitesse et/ou amplitude reflétant le passage d'une forme (respectivement de catégorie à l'autre).

Pour chacun des objets graphiques O1, O2, ..., ON, il est généré un signal de modulation SM1, SM2, ..., SMN correspondant. Un signal de modulation sert à définir les variations en fonction du temps d'un ou plusieurs paramètres d'application de la transformation élémentaire appliquée à l'objet graphique considéré. Par exemple, le degré de transformation di(t) à l'instant t est défini par l'amplitude SMi(t) du signal de modulation SMi à l'instant t.

Dans un ou plusieurs modes de réalisation, un objet graphique animé OA1, OA2, ..., OAN est généré pour chaque objet graphique O1, O2, ..., ON correspondant à partir d'une ou plusieurs transformations élémentaires correspondantes et d'un signal de modulation correspondant. L'objet graphique animé OA1, OA2, ..., OAN ainsi généré est présenté sur un écran d'affichage 105.

Dans un ou plusieurs modes de réalisation, un stimulus visuel est un objet graphique animé OAi (i nombre entier compris entre 1 et N) obtenu par application à un objet graphique correspondant Oi d'une séquence temporelle STi de transformations élémentaires paramétrée temporellement par un signal de modulation correspondant SMi. Ainsi, à chaque instant tz d'une suite discrète d'instants t0, t1, ...tz, ... dans un intervalle de temps [tmin, tmax], un objet graphique modifié OAi(tz) est généré, par application d'une transformation élémentaire Ti correspondante à l'objet graphique Oi avec un degré de transformation di(tz) correspondant à l'amplitude SMi(tz) à l'instant tz du signal de modulation SMi correspondant à l'objet graphique Oi. L'objet graphique animé correspond ainsi à la succession temporelle des objets graphiques modifiés OAi(tz) losrque tz varie dans l'intervalle de temps [tmin, tmax].

Dans un ou plusieurs modes de réalisation, des signaux EEG, notés E1, E2, ..., EX, sont acquis au moyen d'un équipement 130 d'acquisition de signaux EEG. A partir des signaux EEG E1, E2, ..., EX, un signal de modulation reconstruit SMR est généré.

Dans un ou plusieurs modes de réalisation, le signal de modulation reconstruit SMR est généré par application d'un modèle de reconstruction MR aux signaux E1, E2, ..., EX. Les paramètres du modèle de reconstruction MR sont notés P1, P2, ...PK.

Le modèle de reconstruction MR peut être un modèle linéaire, le signal de modulation reconstruit étant une combinaison linéaire des signaux E1, E2, ..., EX.

D'autres modèles plus élaborés peuvent être utilisés, notamment des modèles par réseaux de neurones.

Dans un ou plusieurs modes de réalisation, un signal de modulation est composé de signaux élémentaires. Ces signaux élémentaires peuvent être des signaux rectangulaires, triangulaires, sinusoïdaux, etc. Les signaux de modulation peuvent avoir des durées différentes. Dans un ou plusieurs modes de réalisation, les signaux de modulation sont périodiques, un motif temporel étant reproduit périodiquement par chaque signal de modulation. Un signal de modulation présente par exemple un motif temporel périodique, se répétant avec fréquence comprise entre 2 et 20 Hz, ce signal de modulation étant échantillonné à une fréquence d'échantillonnage correspondant à la fréquence de rafraîchissement (en général, supérieure à 60Hz) de l'écran sur lequel les stimuli visuels (c'est-à-dire les objets graphiques animés) générés à partir des signaux de modulation sont affichés.

L'amplitude du signal de modulation SMi sert à définir un degré de transformation. La relation entre L'amplitude du signal de modulation SMi et le degré de transformation peut être linéaire ou non. L'amplitude d'un signal de modulation SMi peut varier entre une valeur minimale (correspondant à un premier degré de transformation) et une valeur maximale (correspondant à un deuxième degré de transformation).

Dans un ou plusieurs modes de réalisation, les signaux de modulation sont dans ce cas indépendants deux à deux: les signaux de modulation sont composés de sorte que la dépendance, mesurée dans le domaine temporel et/ou fréquentiel, entre deux signaux de modulation distincts quelconques soit minimale (par exemple nulle) ou inférieure à un seuil SC1 donné. La dépendance entre deux signaux peut être quantifiée par un degré de dépendance statistique. Le degré de dépendance statistique entre deux signaux de modulation peut être calculé, dans le domaine temporel, par exemple par un coefficient de corrélation temporelle et/ou, dans le domaine fréquentiel, par exemple par le taux de cohérence spectrale.

Dans un ou plusieurs modes de réalisation, pour chaque paire de signaux de modulation correspondant à des stimuli visuels distincts, les signaux de modulation sont décorrélés temporellement deux à deux.

Dans un ou plusieurs modes de réalisation, un degré de dépendance statistique peut être calculé pour chaque paire de signaux de modulation correspondant à des stimuli visuels distincts, puis un degré de dépendance statistique global (par exemple, le degré de dépendance moyen, le degré de dépendance maximal ou le degré de dépendance cumulé) peut être calculé pour toutes les paires de signaux de modulation. La détermination des signaux de modulation est effectuée en cherchant des signaux de modulation qui minimisent ce degré de dépendance statistique global ou permettent d'obtenir un degré de dépendance statistique global inférieur à un seuil SC1 prédéterminé.

Dans un ou plusieurs modes de réalisation, le degré de dépendance statistique calculé pour chaque paire de signaux de modulation correspondant à des stimuli visuels distincts est nul ou inférieur à un seuil SC1.

Dans un ou plusieurs modes de réalisation, le degré de dépendance statistique entre deux signaux de modulation peut être calculé comme le coefficient de corrélation temporelle entre ces signaux, où le coefficient de corrélation $\rho(X,Y)$ entre deux signaux X et Y peut être obtenu par la formule de Pearson :

$$\rho(X, Y) = \frac{\mathbb{E}\big[\big(X - \mathbb{E}(X)\big)\big(Y - \mathbb{E}(Y)\big)\big]}{\sigma_X \, \sigma_Y} = \frac{\mathbb{E}[XY] - \mathbb{E}[X]\mathbb{E}[Y]}{\sigma_X \, \sigma_Y}$$

où $\mathbb{E}$ désigne l'espérance mathématique d'un signal, et $\sigma$ son écart type. Le coefficient de corrélation temporelle est compris entre 0 et 1, la valeur 0 correspondant à des signaux décorrélés temporellement.

Le degré de dépendance statistique entre les signaux de modulation peut être déterminé par d'autres critères mathématiques, telles que la corrélation de Spearman ou le taux de Kendall, ou remplacée par des mesures de dépendance statistique comme l'information mutuelle.

Le taux de cohérence spectrale entre deux signaux x (t) et y (t) est une fonction à valeurs réelles qui peut être définie par exemple par le rapport

$$|Gxy\ (f)|^2 / (Gxx\ (f)\ *\ Gyy\ (f))$$

où Gxy (f) est la densité spectrale croisée entre x et y, et Gxx (f) et Gyy (f) la densité autospectrale de x et y respectivement.

Dans un ou plusieurs modes de réalisation, le degré de dépendance statistique est calculé sur une période de référence, correspondant par exemple à la durée de la fenêtre de reconstruction (voir étape 414) et/ou la fenêtre de décodage (voir étape 415).

Une discrimination efficace des signaux de modulation est possible lorsque le degré de dépendance statistique global (calculé par exemple comme le degré de dépendance moyen, le degré de dépendance maximal ou un degré de dépendance cumulé), calculé sur toutes les paires de signaux de modulation correspondant à des stimuli visuels distincts, est nul (par exemple pour des signaux décorrélés temporellement) ou inférieur à un seuil SC1, choisi par exemple égal à 0,2 (soit 20% respectivement si ce degré est exprimé en pourcentage). Plus le degré de dépendance statistique global est faible, plus l'identification du stimulus visuel observé par un individu attentif à ce stimulus est efficace et facile. La probabilité d'erreur de discrimination (correspondant au pourcentage de cas dans lesquels le stimulus visuel identifié à l'étape 415 n'est pas celui vers lequel l'individu porte effectivement son attention visuelle) parmi l'ensemble des signaux de modulation, du signal de modulation qui a servi à générer le stimulus visuel observé par le sujet, est également d'autant plus faible. Le seuil SC1 peut dépendre du choix du type de signaux de modulation. En pratique, on peut fixer une probabilité maximale d'erreur de discrimination (une probabilité acceptable pour une application donnée par exemple), et ajuster les signaux de modulation de sorte à rester en dessous de ce taux maximal d'erreur de discrimination. On comprend ici que, même dans le cas où la reconstruction (voir étape 414) est idéale (c'est-à-dire que le signal reconstruit est égal à chaque instant à l'un des signaux de modulation SMi), la qualité du décodage (voir étape 415) dépend du degré de dépendance statistique entre les signaux de modulation, car si les signaux de modulation SMi sont entièrement dépendants eux, il sera impossible de sélectionner un signal de modulation SMi plutôt qu'un autre.

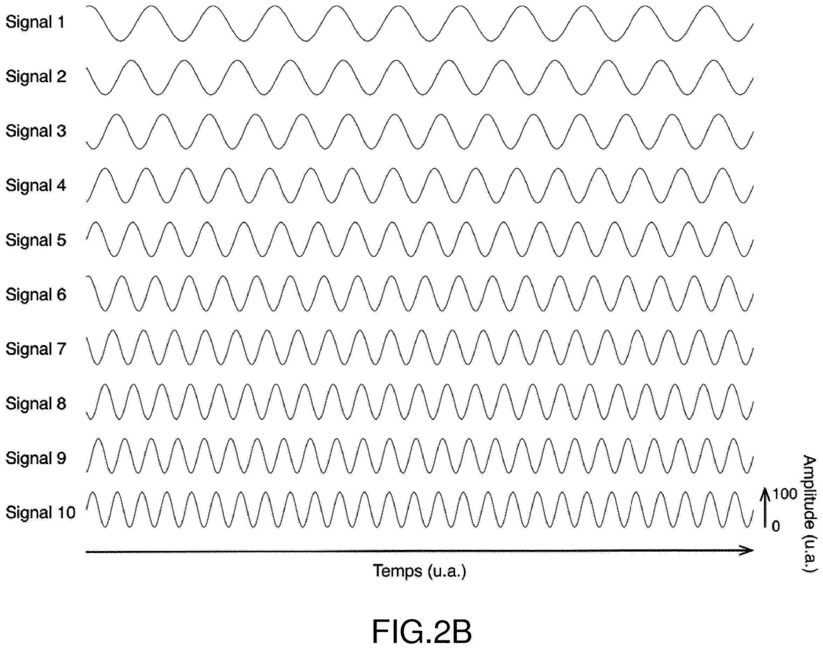

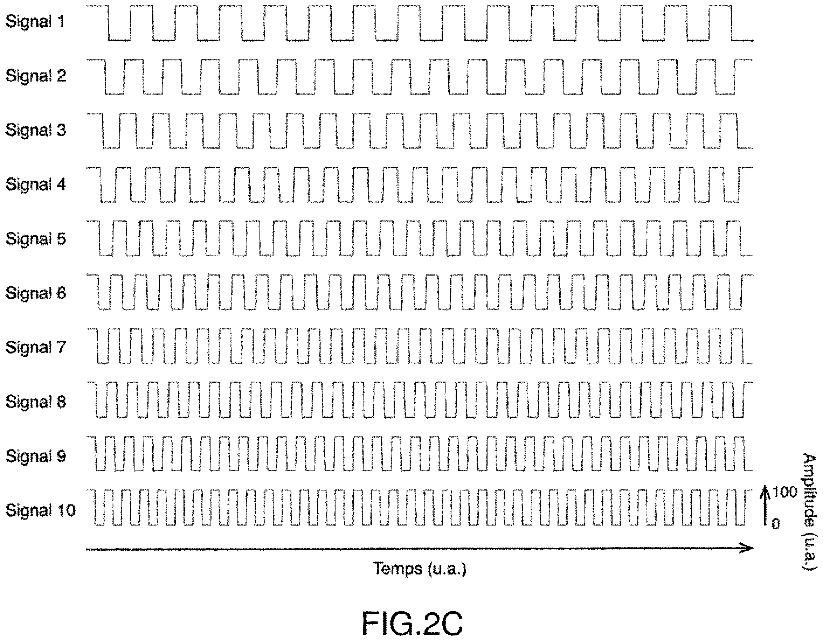

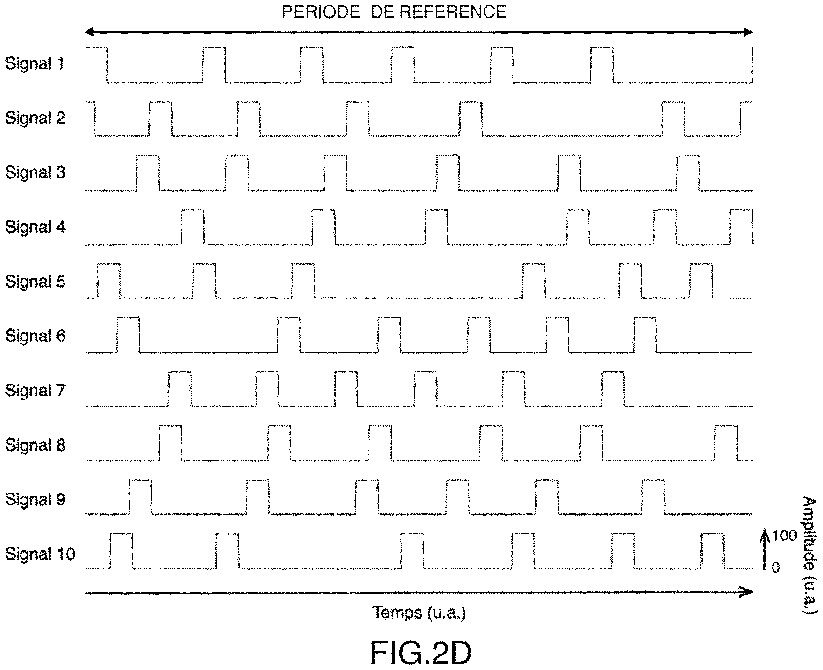

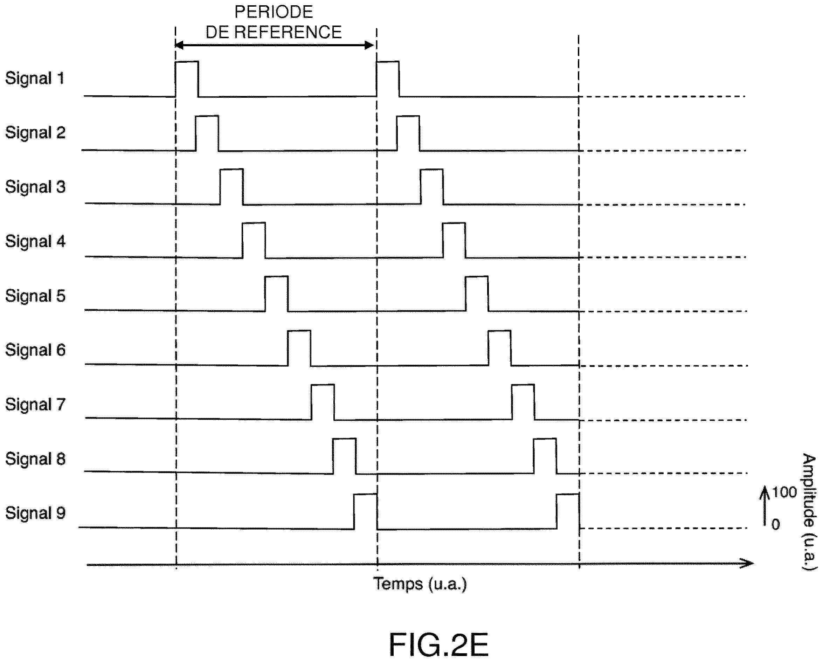

Chacune des FIGS. 2B, 2C, 2D, 2E représente de manière schématique un ensemble de signaux de modulation utilisables dans un système de détermination de la focalisation de l'attention visuelle pour la génération de stimuli visuels.

Dans la figure 2B, est représenté un premier ensemble d'exemple de 10 signaux de modulation, signal 1 à signal 10, qui sont décorrélés temporellement deux à deux. Ces 10 signaux sont des signaux sinusoïdaux, périodiques, ayant des fréquences (et donc périodes) différentes entre 1 Hz et 2 Hz par pas de 0.2 Hz, de telle sorte que deux signaux quelconques pris dans ce premier ensemble n'ont pas la même fréquence. Les phases de ces signaux peuvent être quelconques. Dans l'exemple représenté à la FIG. 2B, l'amplitude de ces signaux varie entre 0% et 100% signifiant que le degré de transformation correspondant varie (avec un coefficient de proportionnalité adapté) entre une valeur minimale et une valeur maximale. Pour toutes les paires de signaux de modulation de cet ensemble de 10 signaux, le taux de recouvrement spectral est nul (i.e. absence de composante fréquentielle commune) et le coefficient de corrélation temporelle maximal sur toutes les paires de signaux de modulation est de 0,2, ce coefficient de corrélation temporel étant calculé sur une fenêtre de corrélation de 4 secondes.

Dans la figure 2C, est représenté un deuxième ensemble d'exemple de 10 signaux de modulation, signal 1 à signal 10, qui sont décorrélés temporellement deux à deux. Ces 10 signaux sont des signaux rectangulaires, périodiques, ayant des fréquences (et donc périodes) différentes entre 1 Hz et 2 Hz par pas de 0.2 Hz, de telle sorte que deux signaux quelconques pris dans ce deuxième ensemble n'ont pas la même fréquence. Les phases de ces signaux peuvent être quelconques. Comme pour la FIG. 2B, l'amplitude de ces signaux varie entre 0% et 100% signifiant que le degré de transformation correspondant varie (avec un coefficient de proportionnalité adapté) entre une valeur minimale et une valeur maximale. Pour toutes les paires de signaux de modulation de cet ensemble de 10 signaux, le taux de recouvrement spectral peut être non nul si certaines composantes harmoniques sont communes mais le coefficient de corrélation temporelle maximal sur toutes les paires de signaux de modulation est de 0,17, ce coefficient de corrélation temporelle étant calculé sur une fenêtre de corrélation de 4 secondes.

Dans la figure 2D, est représenté un troisième ensemble d'exemple de 10 signaux de modulation, signal 1 à signal 10, qui sont décorrélés temporellement deux à deux. Ces 10 signaux sont des signaux périodiques ayant la même période (appelée période de référence sur la FIG. 2D) et sont composés de signaux rectangulaires élémentaires de telle sorte que les motifs temporels de deux signaux quelconques pris dans ce troisième ensemble sont distincts au cours de la période de référence. Dans ce cas, la phase de chacun des signaux est importante en ce qu'elle doit être ajustée de sorte à limiter le coefficient de corrélation temporelle, et donc le degré de dépendance statistique, à une valeur maximale pour chaque paire de signaux de modulation distincts sélectionnée dans cet ensemble de 10 signaux de modulation. Comme pour la FIG. 2B, l'amplitude de ces signaux varie entre 0% et 100% signifiant que le degré de transformation correspondant varie (avec un coefficient de proportionnalité adapté) entre une valeur minimale et une valeur maximale.

Dans la figure 2E, est représenté un troisième ensemble d'exemple de 9 signaux de modulation, signal 1 à signal 9, qui sont décorrélés temporellement deux à deux. Ces signaux sont des signaux périodiques ayant la même période (appelée période de référence sur la FIG. 2E). Chacun des signaux de modulation comprend un motif temporel composé d'une courte impulsion rectangulaire d'amplitude 100% suivi d'un signal de plus longue durée d'amplitude 0%, les impulsions rectangulaires des différents signaux de modulation visuels étant décalées dans le temps les unes par rapport aux autres de sorte qu'à un instant donné, un seul signal de modulation a une amplitude à 100% alors que les autres ont une amplitude à 0%. Ces signaux de modulation présentent tous le même motif temporel (avec un déphasage différent), c'est en ajustant la phase de chacun des signaux que l'on peut contrôler le coefficient de corrélation temporelle, et donc le degré de dépendance statistique, entre deux signaux. Lorsque la fonction de transformation utilisée est une fonction de changement de luminosité, l'objet graphique étant visible (luminosité inchangée) quand le signal de modulation est à 100% et invisible lorsque le signal de modulation est à 0% (luminosité nulle), les objets graphiques animés, qui sont obtenus à partir de ces signaux de modulation et de cette transformation élémentaire obtenus, clignotent en apparaissant et disparaissant dans un ordre donné, un seul stimulus visuel étant visible à un instant donné. Pour toutes les paires de signaux de modulation de cet ensemble de 10 signaux, le coefficient de corrélation temporelle est nul.

On peut donc obtenir des signaux de modulation décorrélés temporellement, en utilisant des fréquences, phases ou motifs temporels distincts pour chaque paire de signaux de modulation, par exemple :

- Avec des signaux composés du même motif temporel périodique, mais ayant des fréquences et donc périodes différentes (cas des figures 2B et 2C), peu importe la phase;
- Avec des signaux composés de motifs temporels distincts périodiques, ayant ou non la même durée (i.e. la période du signal), avec des phases spécifiques propres à chaque motif temporel (cas de la figure 2D);
- Avec des signaux composés du même motif temporel périodique, ayant la même période, mais les motifs étant déphasés les uns par rapport aux autres (cas de la figure 2E).

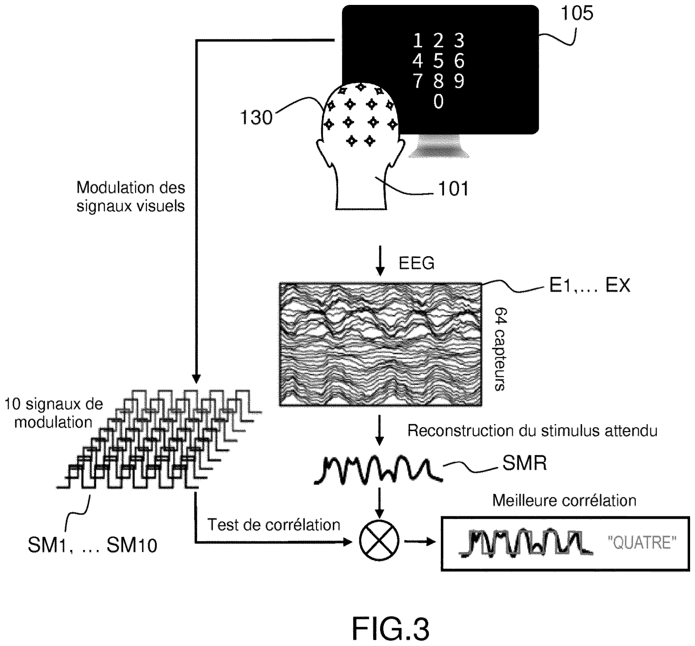

La FIG. 3 représente de manière schématique des aspects d'un procédé et système de détermination de la focalisation de l'attention visuelle.

Dans un ou plusieurs modes de réalisation, le signal de modulation reconstruit SMR est comparé à chacun des signaux de modulation SM1, SM2, ..., SMN pour rechercher un signal de modulation pour lequel le degré de dépendance statistique est maximal. Par exemple, si le degré de dépendance statistique est maximal pour le signal de modulation SM4, cela signifie que l'attention visuelle d'un individu est focalisée sur le stimulus visuel OA4 généré à partir de ce signal de modulation SM4.

Dans l'exemple représenté à la FIG. 3, les stimuli visuels sont les chiffres 0 à 9, l'attention visuelle de l'individu est focalisée sur le chiffre 4 correspondant au stimulus visuel OA4. Le degré de dépendance statistique maximal est trouvé avec le signal de modulation SM4 correspondant.

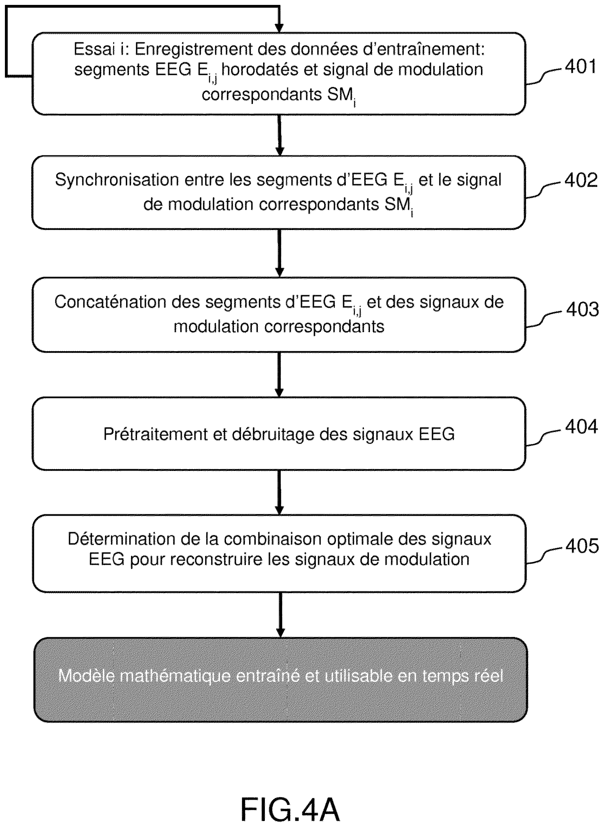

Un exemple de réalisation d'un procédé de génération d'un modèle de reconstruction MR est illustré schématiquement par la figure 4A. Bien que les étapes de ce procédé soient présentées de manière séquentielle, certaines au moins de ces étapes peuvent être omises ou bien être exécutées dans un ordre différent ou bien être exécutées en parallèle ou encore combinées pour ne former qu'une seule étape.

Lors d'une étape 401, un essai i ($i \in [1; N]$) est réalisé avec un stimulus visuel généré à partir d'un signal de modulation SMi : le stimulus visuel est présenté sur un écran d'affichage et un individu est invité à observer le stimulus visuel, c'est-à-dire à porter son attention visuelle sur ce stimulus visuel. Chaque stimulus visuel est un objet graphique animé obtenu par application à un objet graphique d'une séquence temporelle de transformations élémentaires paramétrée temporellement par un signal de modulation correspondant. Des signaux EEG de test Ei,j sont enregistrés pendant que l'individu porte son attention sur le stimulus visuel considéré, où i est l'indice identifiant l'essai et le signal de modulation correspondant, et j l'indice identifiant le canal EEG enregistré. Chacun de ces signaux EEG Ei,j est composé d'une pluralité de segments d'EEG Ei,j,k, où k est l'indice identifiant le segment.

Dans un exemple de mise en oeuvre de l'étape 401, dix stimuli visuels sous forme de chiffres clignotants (chiffres allant de 0 à 9) sont affichés sur un écran, chacun clignotant à une fréquence légèrement différente. L'individu est équipé d'un casque EEG et visionne un écran d'affichage sur lequel les dix chiffres clignotent à des fréquences différentes. Une succession d'essais est réalisée. Chaque essai dure par exemple une dizaine de secondes, l'intervalle entre deux essais étant par exemple de 1 ou 2 secondes. A chaque essai, l'individu a pour instruction de porter son attention sur l'un des chiffres et doit ignorer les autres jusqu'à l'essai suivant. L'individu bascule ainsi son attention d'un stimulus à un autre et génère dans des signaux EEG E1, E2, ..., EX à des fréquences différentes selon le focus d'attention visuelle.

Dans une première variante de réalisation, un horodatage des segments d'EEG est effectué lors de l'étape 401. Un horodatage des signaux de modulation est également effectué lors de l'étape 401. L'horodatage peut être effectué par toute méthode.

Dans cette première variante de réalisation, l'horloge de l'équipement d'acquisition 130 est utilisée pour horodater les segments d'EEG et produire une estampille temporelle $t_i'$ (ou « timecode » selon la terminologie anglo-saxonne) pour chaque segment d'EEG. L'horloge du dispositif de commande 140 est utilisée pour horodater les signaux de modulation et produire une estampille temporelle à chaque fois qu'un événement prédéterminé a lieu dans la stimulation (correspondant par exemple à l'apparition d'un nouveau stimulus visuel à l'écran ou au début de l'affichage d'un stimulus).

Dans une deuxième variante de réalisation, un canal EEG additionnel est utilisé, via lequel des courtes impulsions électriques d'amplitude connue sont transmises à chaque fois qu'un événement prédéterminé a lieu dans la stimulation (correspondant par exemple à l'apparition d'un nouveau stimulus visuel à l'écran ou au début de l'affichage d'un stimulus). Ce canal EEG additionnel contenant les courtes impulsions est sauvegardé avec les segments de données EEG.

L'étape 401 est répétée plusieurs fois, pour chaque stimulus visuel d'une pluralité de stimuli visuels, de sorte à enregistrer les signaux EEG correspondants produits par l'individu lorsqu'il porte son attention visuelle sur le stimulus visuel considéré.

Lors d'une étape 402, l'alignement temporel (ou synchronisation) entre les segments EEG Ei,j,k et les signaux de modulation SMi est effectué. Cette synchronisation peut être effectuée par toute méthode.

Cette synchronisation peut utiliser le double horodatage des segments EEG et des signaux de modulation, ou bien le canal EEG additionnel.

Lorsqu'on utilise le double horodatage, et que les estampilles temporelles sont produites par deux horloges différentes, il est nécessaire de corriger ces valeurs de manière à obtenir des horodatages virtuellement réalisés par la même horloge de référence de sorte à corriger d'éventuelles dérives temporelles entre les horloges. Par exemple, lorsqu'on utilise l'horloge du dispositif de commande comme horloge de référence, les estampilles temporelles $t_i'$ des segments EEG horodatés Ei,j,k, réalisés par l'horloge du dispositif d'acquisition, sont resynchronisés par rapport à l'horloge de référence pour obtenir des estampilles temporelles $t_i$. En associant ces estampilles temporelles corrigées des segments EEG à celles produites pour les signaux de modulation, on peut réaliser l'alignement entre les segments EEG Ei,j,k et les signaux SMi.

La différence entre l'horloge de référence (*t*) du dispositif de commande 140 et celle de l'équipement 130 d'acquisition des données EEG (*t'*) est modélisée par une équation linéaire : $diff = a*(t'-t_0) + b = t' - t$, où *a* représente la dérive entre les deux horloges et *b* l'offset à $t' = t_0$. Pour estimer ces coefficients *a* et *b*, on fait, préalablement à l'étape 401, l'acquisition d'une série de *x* points (t', diff(t')), puis on estime les coefficients *a* et *b* par la méthode des moindres carrés. Afin de pallier les variations aléatoires des temps d'exécution des instructions et de transmission des données entre le dispositif de commande 140 et l'équipement d'acquisition 130, chaque point (t', diff(t')) est obtenu à partir de l'envoi successif de *n* estampilles temporelles $t_k$ par le dispositif de commande 140 vers l'équipement 130 d'acquisition qui produit à chaque réception une estampille $t_k'$. Le point (t', diff(t')) retenu pour le calcul des coefficients *a* et *b* correspond au couple $(t_k', diff = t_k' - t_k)$ qui présente la différence $(t_k' - t_k)$ minimale. Une fois obtenus les coefficients *a* et *b,* les estampilles temporelles $t_i'$ sont corrigées de la façon suivante :

$$t_i = t_i' - a * (t_i' - t_0) - b$$

Les étapes d'horodatage et de recalage temporel sont toutefois optionnelles et ne sont notamment pas nécessaires lorsque l'équipement d'acquisition 130 et le dispositif de commande fonctionnent sur la base de la même horloge.

Lors d'une étape 403, les segments EEG Ei,j,k, sont concaténés de sorte à générer les signaux EEG Ei,j.

Lors d'une étape 404, un prétraitement et débruitage peut être appliqué aux signaux EEG de sorte à optimiser le rapport signal / bruit. Les signaux EEG peuvent en effet être considérablement contaminés par des artéfacts d'origine aussi bien intra- que extra-cérébrale, par exemple des artéfacts électriques, tels que le 50 Hz, courant du réseau électrique en Europe; ou des artéfacts biologiques, tels que les mouvements oculaires, l'électrocardiogramme, l'activité musculaire, etc.). Dans un ou plusieurs modes de réalisation, les signaux E1, E2, ..., EX sont ainsi débruités préalablement à la génération du signal de modulation reconstruit SMR. Ce débruitage peut consister à simplement filtrer les hautes fréquences dans les signaux E1, E2, ..., EX, par exemple toutes les fréquences supérieures à 40 Hz pour éliminer le bruit électrique produit par le réseau électrique. Des approches statistiques multivariées peuvent être utilisées, notamment l'analyse en composantes principales (PCA, Principal Components Analysis), l'analyse en composantes indépendantes (ICA, Independent Components Analysis), et l'analyse de corrélation canonique (CCA, Canonical Corrélation Analysis), permettant de séparer les composantes du signal EEG utiles (provenant de l'activité cérébrale liée à la tâche cognitive en cours), des composantes non-pertinentes.

Lors d'une étape 405, les paramètres du modèle de reconstruction sont déterminés. Cette détermination peut être faite de sorte à minimiser l'erreur de reconstruction. Le modèle de reconstruction comprend par exemple une pluralité de paramètres de combinaison de signaux EEG. Ces paramètres de combinaison sont déterminés par une méthode de résolution d'équations mathématiques de sorte à déterminer des valeurs de paramètres de combinaison optimales, c'est-à-dire les valeurs pour lesquelles l'application du modèle de reconstruction à la pluralité de signaux EEG $E_{i,j}$ de tests enregistrés pour un stimulus visuel permet de générer un signal de modulation reconstruit approchant au mieux le signal de modulation correspondant au stimulus visuel considéré, c'est-à-dire les valeurs pour lesquelles l'erreur de reconstruction est minimale.

Dans un ou plusieurs modes de réalisation, les valeurs $\alpha_j$ des paramètres de combinaison peuvent être fixes (indépendantes du temps). Dans d'autres modes de réalisation, ces valeurs peuvent être ajustées en temps-réel afin de tenir compte d'une éventuelle adaptation de l'activité cérébrale de l'utilisateur 101, ou d'une évolution du rapport signal / bruit dans le signal EEG au cours d'une session d'enregistrement.

Le modèle de reconstruction MR peut être un modèle linéaire, produisant un signal de modulation par combinaison linéaire des signaux $E_{i,j}$. Dans ce cas, les paramètres de combinaison sont des paramètres $\alpha_j$ de combinaison linéaire et les équations mathématiques sont des équations linéaires de la forme :

$$SMi = \sum_j \alpha_j Ei,j \quad \text{pour } i \in [1; N]$$

D'autres modèles plus élaborés peuvent être utilisés, notamment des modèles par réseaux de neurones, pour lesquels le signal de modulation est obtenu en appliquant en cascade d'opérations mathématiques non-linéaires aux signaux $E_{i,j}$. Par exemple, un réseau siamois, pour lequel un réseau de neurones est entraîné (à partir de données de calibration) à faire correspondre à tout signal EEG E un signal temporel unidimensionnel R (en l'occurrence, un signal de modulation) de façon à ce que pour deux signaux EEG E1 et E2 enregistrés à des instants différents, produisent respectivement des signaux unidimensionnels R1 et R2 (en l'occurrence, des signaux de modulation) qui soient similaires lorsque l'attention de l'individu est focalisée sur le même objet graphique animé, et dissimilaires lorsque l'attention de l'individu est focalisée sur deux objets graphiques animés distincts. La notion de similarité entre deux signaux est définie au sens mathématique (il peut s'agir par exemple d'une simple corrélation) et correspond à une fonction qui quantifie le degré de similarité entre deux objets (voir par exemple la page : https://en.wikipedia.org/wiki/Similarity_measure). Plusieurs définitions mathématiques de la similarité peuvent être utilisées, comme par exemple l'opposé de la distance Euclidienne, ou encore la « similarité cosinus » (voir par exemple la page : https://fr.wikipedia.org/wiki/Similarité_cosinus).

Le signal de modulation reconstruit est le signal unidimensionnel R généré par le réseau de neurones à partir d'un échantillon d'EEG E nouvellement acquis.

Dans un ou plusieurs modes de réalisation, les étapes du procédé de génération d'un modèle de reconstruction sont mises en oeuvre par un système 100 selon la figure 1A, par exemple par le dispositif 120 de traitement de signaux.

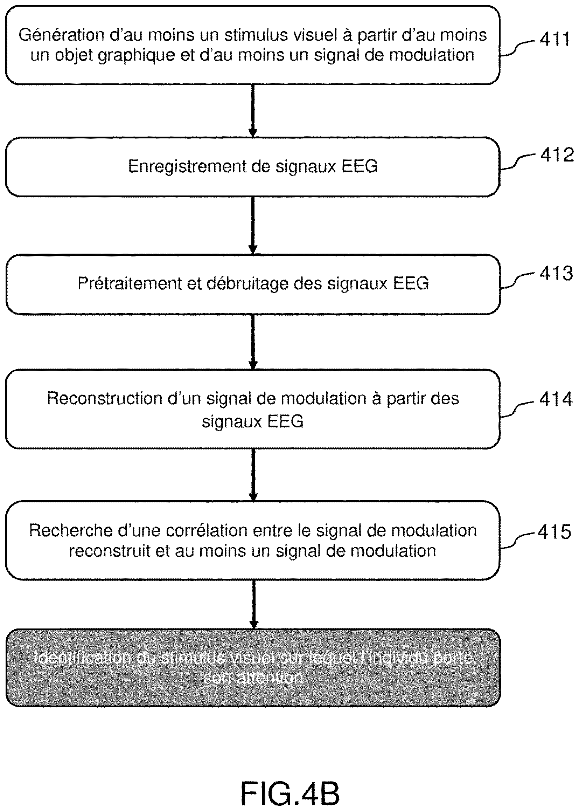

Un exemple de réalisation d'un procédé de détermination de la focalisation de l'attention visuelle d'un individu est illustré schématiquement par la FIG. 4B. Bien que les étapes de ce procédé soient présentées de manière séquentielle, certaines au moins de ces étapes peuvent être omises ou bien être exécutées dans un ordre différent ou bien être exécutées en parallèle ou encore combinées pour ne former qu'une seule étape.

Dans un ou plusieurs modes de réalisation, les étapes du procédé de détermination de la focalisation de l'attention visuelle sont mises en oeuvre par un système 100 selon la figure 1A, par exemple par le dispositif 120 de traitement de signaux et le dispositif 110 de génération de signaux d'affichage.

Lors d'une étape 411, le dispositif de génération de signaux d'affichage 110 est configuré pour générer une pluralité de stimuli visuels à partir d'une pluralité d'objets graphiques O1, O2, ..., ON, d'une pluralité de transformations élémentaires T1, T2, ..., TP et d'une pluralité de signaux de modulation SM1, SM2, ..., SMN. Un stimulus visuel est un objet graphique animé OAi (i compris entre 1 et N) obtenu par application à un objet graphique correspondant Oi d'une séquence temporelle STi de transformations élémentaires paramétrée temporellement par un signal de modulation correspondant SMi.

Dans un ou plusieurs modes de réalisation, le nombre N de stimuli visuels, de signaux de modulation et d'objets graphiques est égal à 1.

Dans un ou plusieurs modes de réalisation, le nombre P de transformations élémentaires est égal à 1. Chaque transformation élémentaire de la séquence temporelle STi de transformations élémentaires peut ainsi correspondre à une même transformation élémentaire dont un paramètre d'application varie au cours du temps.

Au cours du temps, l'individu peut porter son attention visuelle d'un objet graphique animé à un autre. Pendant ce temps, lors d'une étape 412, les signaux électroencéphalographiques E1, E2, ..., Ej, ..., EX produits par l'individu sont enregistrés par l'équipement d'acquisition 130.

Dans un ou plusieurs modes de réalisation, le dispositif de traitement de signaux 120 est configuré pour obtenir une pluralité de signaux électroencéphalographiques E1, E2, ..., Ej, ..., EX produits par l'individu focalisant son attention vers l'un des stimuli visuels OAi.

Lors d'une étape 413, les signaux électroencéphalographiques E1, E2, ...Ej, ...EX sont prétraités et débruités de sorte à améliorer la fiabilité du procédé de détermination de la focalisation de l'attention visuelle. Le prétraitement peut consister à synchroniser les segments de signaux électroencéphalographiques E1, E2, ...Ej, ...EX par rapport à une horloge de référence, comme expliqué ci-dessus pour l'étape 402, à concaténer les segments de signaux électroencéphalographiques comme expliqué ci-dessus pour l'étape 403, et/ou à effectuer un débruitage des signaux électroencéphalographiques comme expliqué ci-dessus pour l'étape 404.

Dans un ou plusieurs modes de réalisation, le dispositif de traitement de signaux 120 est configuré pour, lors d'une étape 414, obtenir un signal de modulation reconstruit SMR par reconstruction d'un signal de modulation à partir pluralité de signaux électroencéphalographiques E1, E2, ..., Ej, ..., EX .

Dans un ou plusieurs modes de réalisation, le dispositif de traitement de signaux 120 est configuré pour reconstruire un signal de modulation et générer un signal de modulation reconstruit SMR à partir de la pluralité de signaux électroencéphalographiques obtenus à l'étape 413 ou 412 (avec ou sans prétraitement et/ou débruitage). Dans un ou plusieurs modes de réalisation, la reconstruction est effectuée par application d'un modèle de reconstruction à la pluralité de signaux électroencéphalographiques obtenus à l'étape 413 ou 412 (avec ou sans prétraitement et/ou débruitage). Cette reconstruction peut être effectuée sur une fenêtre temporelle glissante donnée, appelée ici fenêtre de reconstruction, et répétée périodiquement pour chaque position temporelle de la fenêtre de reconstruction.

Par exemple, lorsque le modèle de reconstruction MR est un modèle linéaire, produisant un signal de modulation par combinaison linéaire des signaux E1, E2, ..., Ej, ..., EX. Dans ce cas, les paramètres de combinaison sont les paramètres $\alpha_j$ de combinaison linéaire obtenus lors de l'étape 405 et le signal de modulation reconstruit SMR est calculé par combinaison linéaire des signaux E1, E2, ..., Ej, ..., EX:

$$\mathrm{SMR} = \sum_j \; \alpha_j \mathrm{Ej}$$

Dans un ou plusieurs modes de réalisation, le dispositif de traitement de signaux 120 est configuré pour, lors d'une étape 415 (appelée étape de décodage), calculer un degré de dépendance statistique entre le signal de modulation reconstruit et chaque signal de modulation de l'ensemble des signaux de modulation et identifier au moins stimulus visuel correspondant à un signal de modulation pour lequel le degré de dépendance statistique est supérieur à un seuil SC2, de valeur comprise par exemple entre 0,2 et 0,3. Le fait d'identifier d'au moins stimulus visuel correspondant à un signal de modulation pour lequel le degré de dépendance statistique est supérieur à un seuil SC2 signifie que l'attention visuelle de l'individu vient, a priori, de se porter vers ce stimulus visuel et/ou que ce ou ces stimuli visuels viennent d'apparaître dans une zone de l'écran d'affichage observée par l'individu. On peut donc de servir de cette identification pour détecter qu'un changement d'affichage a eu lieu et/ou qu'un changement de focus d'attention visuelle a eu lieu. Le degré de dépendance statistique peut être déterminé comme décrit plus haut dans ce document. Le degré de dépendance statistique est par exemple un coefficient de corrélation temporelle entre le signal de modulation reconstruit et un signal de modulation de l'ensemble des signaux de modulation.

Dans un ou plusieurs modes de réalisation, le nombre N de stimuli visuels, de signaux de modulation et d'objets graphiques est supérieur strictement à 1 et le dispositif de traitement de signaux 120 est configuré pour, en outre, lors d'une étape 415 rechercher, parmi la pluralité de signaux de modulation SM1, SM2, ..., SMN, le signal de modulation SMi pour lequel le degré de dépendance statistique avec le signal de modulation reconstruit SMR est maximale et identifier le stimulus visuel OAi correspondant au signal de modulation SMi pour lequel le degré de dépendance statistique est maximal. L'attention visuelle est a priori focalisée vers le stimulus visuel OAi identifié. La recherche s'effectue par exemple par calcul d'un degré de dépendance statistique entre le signal de modulation reconstruit SMR et chaque signal de la pluralité de signaux de modulation SM1, SM2, ..., SMN. Cette étape de décodage peut être effectuée sur une fenêtre temporelle glissante donnée, appelée ici fenêtre de décodage, et répétée périodiquement pour chaque position temporelle de la fenêtre de décodage. La durée de la fenêtre de décodage peut être identique à celle de la fenêtre de reconstruction.

Dans un ou plusieurs modes de réalisation, lorsque le nombre N de stimuli visuels, de signaux de modulation et d'objets graphiques est supérieur strictement à 1, un ou plusieurs stimuli visuels pouvant être affichés à un instant donné sur l'écran d'affichage 105. L'étape 415 de décodage peut néanmoins être identique quel que soit le nombre de stimuli visuels affichés à un instant donné, la dépendance statistique pouvant être recherchée avec l'un quelconque des signaux de modulation SM1, SM2, ..., SMN correspondant aux stimuli visuels susceptibles d'être affichés. On s'affranchit ainsi du besoin de modification dynamique et de synchronisation des traitements effectués lors de l'étape 415 de décodage par rapport aux variations du contenu effectivement affiché. Ceci peut être très utile quand les stimuli visuels sont intégrés dans une vidéo ou que l'interface utilisateur, dans laquelle les stimuli visuels sont intégrés, est modifiée dynamiquement au fur et à mesure que l'utilisateur interagit ave cette interface utilisateur.

Dans un exemple de réalisation, dix stimuli visuels sous forme de chiffres clignotants (chiffres allant de 0 à 9) sont affichés sur un écran, chacun clignotant à une fréquence légèrement différente ou avec la même fréquence mais en apparaissant alternativement à l'écran. L'individu est équipé d'un casque EEG et visionne un écran d'affichage sur lequel les dix chiffres clignotent à des fréquences différentes.

Dans un mode de réalisation, lors de la détermination de la focalisation de l'attention visuelle, en cas d'ambiguïté entre deux ou plusieurs stimuli visuels ou en cas de perturbations et/ou artefacts dans les signaux EEG enregistrés du fait par exemple de mouvements de l'utilisateur, on peut modifier temporairement (par exemple, pendant la durée nécessaire pour lever l'ambiguïté ou pour retrouver des signaux moins perturbés) les signaux de modulations des stimuli visuels. Cette modification peut être effectuée de sorte par exemple à n'afficher que les stimuli visuels pour lesquels il y a ambiguïté et/ou à modifier les signaux de modulations des stimuli visuels pour lesquels il y a ambiguïté.

La modification des signaux de modulations peut consister à modifier la fréquence ou le motif temporel des signaux de modulation, de sorte à augmenter la fréquence et/ou la durée totale de visibilité et/ ou le degré de transformation des stimuli visuels pour lesquels il y a ambiguïté et réduire la fréquence et/ou la durée totale de visibilité et/ou le degré de transformation des autres stimuli visuels. La modification peut également consister à permuter les signaux de modulation entre eux, sans changer leur motif temporel ou leur fréquence. Cette permutation peut être aléatoire. Une telle permutation revient, lorsque les signaux de modulation et la transformation élémentaire sont définis de sorte à ce que les stimuli visuels clignotent en apparaissant et disparaissant à l'écran d'affichage dans un ordre donné (voir l'exemple de la FIG. 2E), à modifier, par exemple aléatoirement, l'ordre d'apparition des stimuli visuels, de manière à ce que les stimuli pour lesquels il y a ambiguïté soient visibles plus fréquemment. La permutation peut être combinée avec une modification des signaux de modulations visant à augmenter la fréquence et/ou la durée totale de visibilité et/ ou le degré de transformation des stimuli visuels pour lesquels il y a ambiguïté.

Le dispositif de traitement de signaux 120 permet d'identifier automatiquement, sans autre information que l'EEG, le stimulus visuel sur lequel l'attention est focalisée. Un modèle de reconstruction permet de générer à partir de l'EEG brut, le signal de modulation reconstruit. Le signal de modulation est corrélé aux signaux de modulation correspondants aux différents objets graphiques animés, le stimulus visuel observé étant celui correspondant au signal de modulation pour lequel le degré de dépendance statistique est maximal.

Des tests obtenus en regroupant les résultats de plusieurs individus permettent de montrer que la méthode de détermination du stimulus visuel observé est très robuste (taux d'erreur inférieur à 10% pour des signaux E1, E2, ..., EX enregistrés pendant une durée de 1 seconde), et ce même avec une phase d'apprentissage très courte, de quelques minutes (par exemple, moins de 5 minutes) voire de quelques secondes (par exemple, moins de 5 secondes) mise en oeuvre sur quelques stimuli types.

Le procédé de détermination de la focalisation de l'attention visuelle est applicable non seulement à des chiffres mais à des nombreuses interfaces homme-machine, par exemple à clavier alphanumérique complet à 26 caractères ou plus.

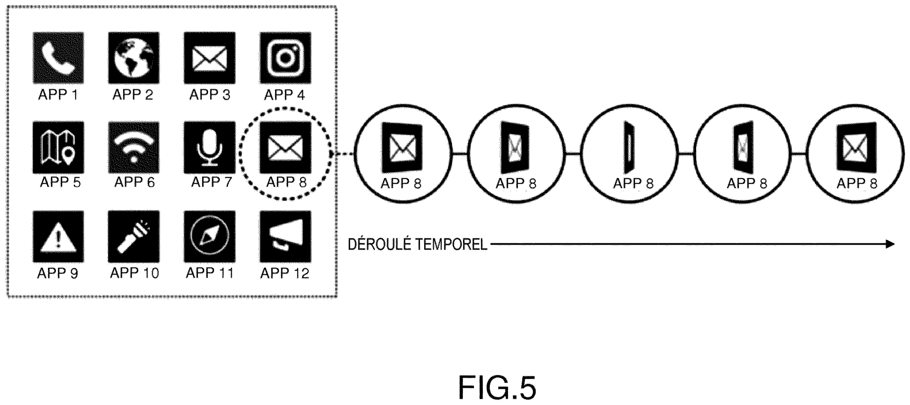

La Fig. 5 illustre un autre exemple d'interface homme-machine auquel ce procédé est applicable. Dans cet exemple, des stimuli dynamiques consistent en des logos ou icones qui sont animés, non pas par des transformations élémentaires qui agissent sur l'intensité lumineuse du logo, mais par application de mouvements de sorte à déplacer le logo sur lui-même, dans un plan ou dans un espace tridimensionnel. Ces mouvements sont par exemple des oscillations ou rotations, à différentes fréquences décodables en temps-réel. Dans ce cas, l'amplitude du signal de modulation correspondant indique le degré de transformation à un instant donné, c'est-à-dire l'angle de rotation à appliquer à un instant donné. Ces mouvements sont par exemples périodiques.

Sur la FIG. 5, 12 logos APP1 à APP12 sont présentés, arrangés en une grille de 4 X 3 logos. Bien que cette figure soit présentée en noir et blanc, les logos peuvent également être en couleur. Dans l'exemple de la FIG. 5, les logos pivotent sur eux-mêmes à différentes fréquences, comme illustré par la FIG. 5 pour le logo APP8. Chacun de ces logos est animé par oscillation en rotation sur lui- même à une fréquence distincte de celle des autres logos. La rotation périodique appliquée aux icônes induit des réponses cérébrales détectables dans les signaux EEG à la fréquence propre de rotation, qui peuvent être décodées en temps réel grâce aux techniques décrites plus haut. Ce type d'interface est hautement flexible et peut notamment être adapté pour smartphone ou tablette. Une telle interface homme-machine permet par exemple de réaliser des interfaces graphiques pour tout type de dispositif informatique, par exemple pour des applications logicielles et/ou systèmes d'exploitation sur un terminal mobile ou ordinateur, que l'écran d'affichage soit un écran tactile ou non.

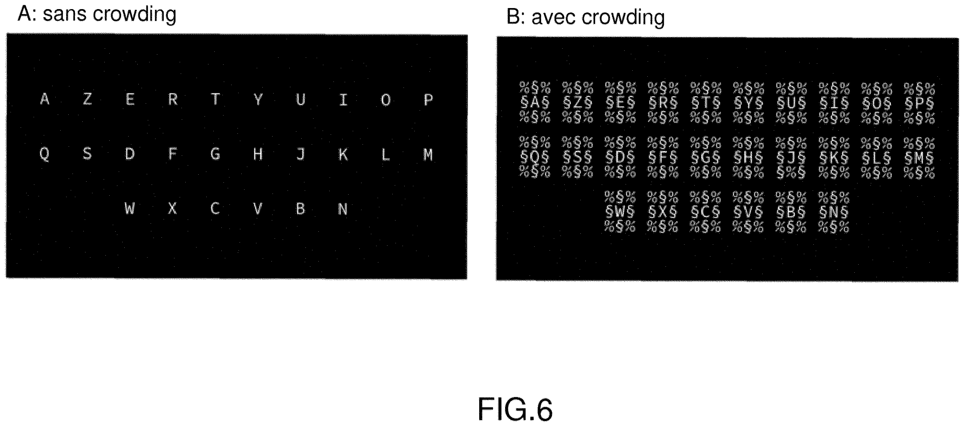

La Fig. 6 illustre un autre exemple d'interface homme-machine utilisable. Afin de faciliter le focus de l'attention visuelle d'un individu vers un stimulus visuel et de minimiser l'influence, sur les signaux EEG, des stimuli visuels voisins, il peut être fait appel à une technique dite d'encombrement ou « crowding ». Cette technique consiste à entourer chaque stimulus visuel de masques latéraux, animés ou non, qui réduisent les perturbations visuelles liées à l'animation des stimuli visuels voisins et permettent à l'individu de focaliser plus efficacement son attention sur un stimulus en particulier, et par conséquent d'en améliorer le décodage.

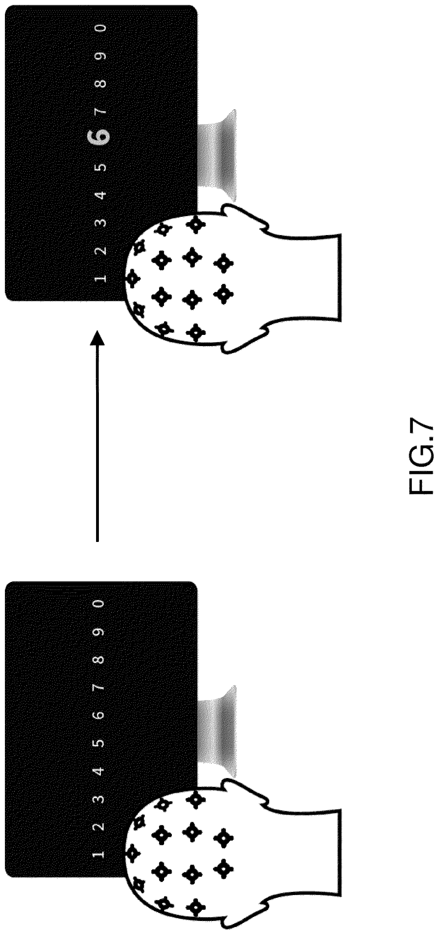

La Fig. 7 illustre un autre exemple d'interface homme-machine dans lequel un feedback est donné à l'utilisateur sur le stimulus visuel ayant été identifié comme étant observé par l'utilisateur. L'interface homme-machine comprend les chiffres 0 à 9. Dans l'exemple de la fig. 7, l'utilisateur observe le chiffre 6 et le feedback consiste à agrandir le chiffre identifié comme étant observé par application d'un procédé de détermination de la focalisation de l'attention visuelle selon la présente description. De manière générale, le feedback donné à l'utilisateur peut consister à mettre en évidence le stimulus visuel identifié, par exemple, par surbrillance, clignotement, zoom, changement de position, changement de taille ou changement de couleur, etc,

Dans un ou plusieurs modes de réalisation, les stimuli visuels font partie d'une interface homme-machine d'une application logicielle ou d'un dispositif informatique, et une commande est envoyée pour déclencher l'exécution d'une ou plusieurs opérations associées au stimulus visuel identifié suite à l'identification du stimulus visuel observé par la mise en oeuvre d'un procédé de détermination de la focalisation de l'attention visuelle selon la présente description.

Dans un ou plusieurs modes de réalisation, les différentes étapes du ou des procédés décrits dans ce document sont mises en oeuvre par un logiciel ou programme d'ordinateur.

La présente description concerne ainsi un logiciel ou programme d'ordinateur comprenant des instructions logicielles ou instructions de code de programme, lisibles et/ou exécutables par un ordinateur ou par un processeur de données, ces instructions étant configurées pour commander l'exécution des étapes d'un ou des procédés décrits dans ce document lorsque ce programme d'ordinateur est exécuté par un ordinateur ou processeur de données.

Ces instructions peuvent utiliser n'importe quel langage de programmation, et être sous la forme de code source, code objet, ou de code intermédiaire entre code source et code objet, tel que dans une forme partiellement compilée, ou dans n'importe quelle autre forme souhaitable. Ces instructions sont destinées à être stockées dans une mémoire d'un dispositif informatique ou système informatique, chargées puis exécutées par une unité de traitement ou processeur de données de ce dispositif informatique ou système informatique afin de mettre en oeuvre les étapes du ou des procédés décrits dans ce document. Ces instructions peuvent être stockées, temporairement ou définitivement, en totalité ou en partie, sur un support lisible par ordinateur, non transitoire, d'un dispositif de stockage local ou distant comportant un ou plusieurs supports de stockage.

La présente description concerne aussi un support de données lisible par un processeur de données, comprenant des instructions d'un logiciel ou programme d'ordinateur tel que mentionné ci-dessus. Le support de données peut être n'importe quelle entité ou dispositif capable de stocker de telles instructions. Des modes de réalisation de supports lisibles par ordinateur comportent, sans s'y limiter, à la fois des supports de stockage informatiques et des supports de communication comportant n'importe quel support qui facilite le transfert d'un programme d'ordinateur d'un endroit à un autre. Un tel support de stockage peut être un dispositif de stockage optique tels que disque compact (CD, CD-R ou CD-RW), DVD (DVD-Rom ou DVD-RW) ou Blu-ray, un support magnétique tel que disque dur, bande magnétique ou disquette, un support de stockage amovible de type clef USB, une carte à mémoire SD ou micro SD, ou encore une mémoire, telle que mémoire vive (RAM), mémoire morte (ROM), mémoire cache, mémoire rémanente, mémoire de sauvegarde (par exemple des mémoires programmables ou flash), etc.

La présente description concerne aussi un dispositif informatique ou système informatique comprenant des moyens de mise en oeuvre des étapes du ou des procédés décrits dans ce document. Ces moyens sont des moyens logiciels (software) et/ou matériels (hardware) de mise en oeuvre des étapes du ou des procédés décrits dans ce document.

La présente description concerne aussi un dispositif informatique ou système informatique comprenant au moins une mémoire de stockage d'instructions de code d'un programme d'ordinateur pour l'exécution de tout ou partie des étapes d'un ou des procédés décrits dans ce document et au moins un processeur de données configuré pour exécuter un tel programme d'ordinateur.

**Revendications**

1. Procédé implementé par ordinateur de détermination de la focalisation de l'attention visuelle d'un individu à partir de signaux électroencéphalographiques, le procédé comprenant

   une génération (411) d'un ensemble d'au moins un stimulus visuel à afficher à partir d'au moins un objet graphique, d'au moins une transformation élémentaire et d'un ensemble d'au moins un signal de modulation, un stimulus visuel étant un objet graphique animé obtenu par application à un objet graphique d'une séquence temporelle de transformations élémentaires paramétrée temporellement par un signal de modulation correspondant;

une reconstruction (414) d'un signal de modulation à partir d'une pluralité de signaux électroencéphalographiques produits par l'individu pour obtenir un signal de modulation reconstruit ;

un calcul (415) d'un degré de dépendance statistique entre le signal de modulation reconstruit et chaque signal de modulation de l'ensemble d'au moins un signal de modulation;

une identification (415) d'au moins un stimulus visuel correspondant à un signal de modulation pour lequel le degré de dépendance statistique est supérieur à un premier seuil.

2. Procédé selon la revendication 1, dans lequel l'ensemble d'au moins un stimulus visuel comprend une pluralité de stimuli visuels et l'ensemble d'au moins un signal de modulation comprend une pluralité de signaux de modulation, le procédé comprenant :

une recherche (415), parmi la pluralité de signaux de modulation, d'un signal de modulation pour lequel un degré de dépendance statistique avec le signal de modulation reconstruit est maximal ; et

une identification (415) du stimulus visuel correspondant au signal de modulation pour lequel le degré de dépendance statistique est maximal ;

les signaux de modulations étant composés de sorte qu'un degré de dépendance statistique global, déterminé dans le domaine temporel et/ou fréquentiel, pour toutes les paires de signaux de modulation correspondant à deux stimuli visuels distincts, soit inférieur à un deuxième seuil.

3. Procédé selon la revendication 1 ou 2, dans lequel la reconstruction est effectuée par application d'un modèle de reconstruction à la pluralité de signaux électroencéphalographiques.

4. Procédé selon la revendication 3, dans lequel le modèle de reconstruction comprend une pluralité de paramètres de combinaison de signaux électroencéphalographiques, le procédé comprenant une détermination de valeurs pluralité de paramètres de combinaison de signaux électroencéphalographiques lors d'une phase initiale d'apprentissage.

5. Procédé selon la revendication 4, le procédé comprenant en outre, lors de la phase initiale d'apprentissage appliquée à un sous-ensemble d'au moins un stimulus visuel parmi la pluralité de stimuli visuels,

une obtention, pour chaque stimulus visuel dudit sous-ensemble d'au moins un stimulus visuel, de signaux électroencéphalographiques de tests produits par l'individu focalisant son attention sur le stimulus visuel considéré; et

une détermination de valeurs optimales de la pluralité de paramètres de combinaison de signaux électroencéphalographiques pour lesquelles l'application du modèle de reconstruction à la pluralité de signaux électroencéphalographiques de tests enregistrés pour un stimulus visuel permet de générer un signal de modulation reconstruit approchant au mieux le signal de modulation correspondant au stimulus visuel considéré.

6. Procédé selon l'une quelconque des revendications précédentes, dans lequel chaque signal de modulation définit des variations en fonction du temps d'au moins un paramètre d'application d'une transformation élémentaire à un objet graphique.

7. Procédé selon la revendication 6, dans lequel un paramètre d'application est un degré de transformation ou une vitesse d'application d'une transformation élémentaire.

8. Procédé selon l'une quelconque des revendications précédentes, dans lequel une transformation élémentaire est une transformation de l'ensemble de transformations constitué par une variation d'intensité lumineuse, une variation de contraste, une transformation colorimétrique, une déformation géométrique, une rotation, une oscillation, un déplacement selon une trajectoire, un changement de forme et un changement d'objet graphique ou une combinaison de transformations choisies dans ledit ensemble de transformations.

9. Programme informatique comprenant des instructions de code de programme pour l'exécution des étapes d'un procédé selon l'une quelconque des revendications précédentes lorsque ledit programme informatique est exécuté par un processeur de données.

10. Système informatique comprenant au moins une mémoire de stockage d'instructions de code d'un programme d'ordinateur configuré pour l'exécution d'un procédé selon l'une quelconque des revendications 1 à 7 et au moins un processeur de données configuré pour exécuter ledit programme d'ordinateur.

11. Système de détermination de la focalisation de l'attention visuelle d'un individu à partir de signaux électroencéphalographiques, le système comprenant

un dispositif (110) de génération de signaux d'affichage configuré pour générer (410) un ensemble d'au moins un stimulus visuel à afficher à partir d'au moins un objet graphique, d'au moins une transformation élémentaire et d'un ensemble d'au moins un signal de modulation, un stimulus visuel étant un objet graphique animé obtenu par application à un objet graphique d'une séquence temporelle de transformations élémentaires paramétrée temporellement par un signal de modulation correspondant;
un dispositif (120) de traitement de signaux configuré pour

- obtenir (411) une pluralité de signaux électroencéphalographiques produits par l'individu;
- obtenir (414) un signal de modulation reconstruit par reconstruction d'un signal de modulation à partir de la pluralité de signaux électroencéphalographiques;
- calculer (415) un degré de dépendance statistique entre le signal de modulation reconstruit et chaque signal de modulation dudit ensemble d'au moins un signal de modulation ; et
- identifier (415) au moins un stimulus visuel correspondant à un signal de modulation pour lequel le degré de dépendance statistique est supérieur à un premier seuil.

12. Système selon la revendication 11, dans lequel l'ensemble d'au moins un stimulus visuel comprend une pluralité de stimuli visuels et l'ensemble d'au moins un signal de modulation comprend une pluralité de signaux de modulation, le dispositif (120) de traitement de signaux étant en outre configuré pour

- rechercher (415), parmi la pluralité de signaux de modulation, le signal de modulation pour lequel le degré de dépendance statistique avec le signal de modulation reconstruit est maximal ;
- identifier (415) le stimulus visuel correspondant au signal de modulation pour lequel le degré de dépendance statistique est maximal ;

les signaux de modulations étant composés de sorte qu'un degré de dépendance statistique global, déterminé dans le domaine temporel et/ou fréquentiel, pour toutes les paires de signaux de modulation correspondant à deux stimuli visuels distincts, soit inférieur à un deuxième seuil.

**Patentansprüche**

1. Computerimplementiertes Verfahren zum Bestimmen des visuellen Aufmerksamkeitsfokus eines Individuums auf der Basis von elektroenzephalographischen Signalen, wobei das Verfahren Folgendes beinhaltet:

Erzeugen (411) eines Satzes von mindestens einem auf der Basis von mindestens einem graphischen Objekt anzuzeigenden visuellen Reiz, mindestens einer Elementartransformation und eines Satzes von mindestens einem Modulationssignal, wobei ein visueller Reiz ein animiertes graphisches Objekt ist, das durch Anwenden einer durch ein entsprechendes Modulationssignal zeitlich parametrisierten zeitlichen Sequenz von Elementartransformationen auf ein graphisches Objekt erhalten wird;
Rekonstruieren (414) eines Modulationssignals auf der Basis von mehreren elektroenzephalografischen Signalen, die von dem Individuum erzeugt wurden, um ein rekonstruiertes Modulationssignal zu erhalten;
Berechnen (415) eines statistischen Abhängigkeitsgrads zwischen dem rekonstruierten Modulationssignal und jedem Modulationssignal des Satzes von mindestens einem Modulationssignal;
Identifizieren (415) mindestens eines visuellen Reiz entsprechend einem Modulationssignal, wobei der statistische Abhängigkeitsgrad über einem ersten Schwellenwert liegt.

2. Verfahren nach Anspruch 1, wobei der Satz von mindestens einem visuellen Reiz mehrere visuelle Reize umfasst und der Satz von mindestens einem Modulationssignal mehrere Modulationssignale umfasst, wobei das Verfahren Folgendes beinhaltet:

Suchen (415), unter den mehreren Modulationssignalen, nach einem Modulationssignal, für das ein statistischer Abhängigkeitsgrad von dem rekonstruierten Modulationssignal maximal ist; und

Identifizieren (415) des visuellen Reizes entsprechend dem Modulationssignal, für das der statistische Abhängigkeitsgrad maximal ist;

wobei die Modulationssignale so zusammengesetzt sind, dass ein in der Zeit- und/oder Frequenzdomäne bestimmter globaler statistischer Abhängigkeitsgrad für alle Paare von Modulationssignalen entsprechend zwei verschiedenen visuellen Reizen unter einem zweiten Schwellenwert liegt.

3. Verfahren nach Anspruch 1 oder 2, wobei die Rekonstruktion durch Anwenden eines Rekonstruktionsmodells auf die mehreren elektroenzephalografischen Signale erfolgt.

4. Verfahren nach Anspruch 3, wobei das Rekonstruktionsmodell mehrere Kombinationsparameter von elektroenzephalographischen Signalen umfasst, wobei das Verfahren das Bestimmen von Werten mehrere Kombinationsparameter von elektroenzephalographischen Signalen in einer anfänglichen Lernphase beinhaltet.

5. Verfahren nach Anspruch 4, wobei das Verfahren ferner in der anfänglichen Lernphase, die auf eine Teilmenge von mindestens einem visuellen Reiz unter den mehreren visuellen Reizen angewendet wird, Folgendes beinhaltet:

Erhalten, für jeden visuellen Reiz der genannten Teilmenge von mindestens einem visuellen Reiz, von elektroenzephalographischen Testsignalen, die von dem Individuum erzeugt werden, das seine Aufmerksamkeit auf den visuellen Reiz richtet; und

Bestimmen optimaler Werte der mehreren Kombinationsparameter von elektroenzephalographischen Signalen, bei denen die Anwendung des Rekonstruktionsmodells auf die für einen visuellen Reiz aufgezeichneten mehreren elektroenzephalographischen Testsignale ein rekonstruiertes Modulationssignal erzeugt, das dem Modulationssignal entsprechend dem betrachteten visuellen Reiz am nächsten kommt.

6. Verfahren nach einem der vorherigen Ansprüche, wobei jedes Modulationssignal zeitabhängige Variationen von mindestens einem Parameter für die Anwendung einer Elementartransformation auf ein grafisches Objekt definiert.

7. Verfahren nach Anspruch 6, wobei ein Anwendungsparameter ein Transformationsgrad oder eine Anwendungsgeschwindigkeit einer Elementartransformation ist.

8. Verfahren nach einem der vorherigen Ansprüche, wobei eine Elementartransformation eine Transformation aus dem Satz von Transformationen ist, der aus einer Lichtintensitätsänderung, einer Kontraständerung, einer Farbtransformation, einer geometrischen Verformung, einer Rotation, einer Oszillation, einer Bewegung entlang einer Bahn, einer Formänderung und einer Änderung des grafischen Objekts oder einer Kombination von aus dem genannten Satz von Transformationen ausgewählten Transformationen besteht.

9. Computerprogramm mit Programmcode-Befehlen zur Ausführung der Schritte eines Verfahrens nach einem der vorherigen Ansprüche, wenn das Computerprogramm von einem Datenprozessor ausgeführt wird.

10. Computersystem mit mindestens einem Speicher zum Speichern von Code-Befehlen eines Computerprogramms, das zum Ausführen eines Verfahrens nach einem der Ansprüche 1 bis 7 konfiguriert ist, und mit mindestens einem zur Ausführung des Computerprogramms konfigurierten Datenprozessor.

11. System zur Bestimmung des visuellen Aufmerksamkeitsfokus eines Individuums auf der Basis von elektroenzephalographischen Signalen, wobei das System Folgendes umfasst:

ein Gerät (110) zum Erzeugen von Anzeigesignalen, konfiguriert zum Erzeugen (410) eines Satzes von mindestens einem auf der Basis von mindestens einem grafischen Objekt anzuzeigenden visuellen Reiz, mindestens einer Elementartransformation und einem Satz von mindestens einem Modulationssignal, wobei ein visueller Reiz ein animiertes grafisches Objekt ist, das durch Anwenden einer durch ein entsprechendes Modulationssignal zeitlich parametrisierten zeitlichen Sequenz von Elementartransformationen auf ein grafisches Objekt erhalten wird;

ein Signalverarbeitungsgerät (120), konfiguriert zum:

- Erhalten (411) mehrerer elektroenzephalografischer Signale, die von dem Individuum erzeugt wurden;
- Erhalten (414) eines rekonstruierten Modulationssignals durch Rekonstruktion eines Modulationssignals auf der Basis der mehreren elektroenzephalographischen Signale;
- Berechnen (415) eines statistischen Abhängigkeitsgrads zwischen dem rekonstruierten Modulationssignal und jedem Modulationssignal des genannten Satzes von mindestens einem Modulationssignal; und
- Identifizieren (415) mindestens eines visuellen Reizes entsprechend einem Modulationssignal, für das der statistische Abhängigkeitsgrad größer als ein erster Schwellenwert ist.

12. System nach Anspruch 11, wobei der Satz von mindestens einem visuellen Reiz mehrere visuelle Reize umfasst und der Satz von mindestens einem Modulationssignal mehrere Modulationssignale umfasst, wobei das Signalverarbeitungsgerät (120) ferner konfiguriert ist zum:

- Suchen (415), unter den mehreren Modulationssignalen, des Modulationssignals, für das der statistische Abhängigkeitsgrad mit dem rekonstruierten Modulationssignal maximal ist;
- Identifizieren (415) des visuellen Reizes entsprechend dem Modulationssignal, für das der statistische Abhängigkeitsgrad maximal ist;

wobei die Modulationssignale so zusammengesetzt sind, dass ein in der Zeit- und/oder Frequenzdomäne für alle Paare von Modulationssignalen entsprechend zwei verschiedenen visuellen Reizen bestimmter globaler statistischer Abhängigkeitsgrad kleiner als ein zweiter Schwellenwert ist.

## Claims

1. Method implemented by a computer for determining the focus of an individual's visual attention based on electroencephalographic signals, the method comprising

generating (411) a set of at least one visual stimulus to be displayed from at least one graphical object, from at least one elementary transformation and from a set of at least one modulation signal, a visual stimulus being an animated graphical object obtained by applying to a graphical object a temporal sequence of elementary transformations that is temporally parameterised by a corresponding modulation signal;
reconstructing (414) a modulation signal from a plurality of electroencephalographic signals produced by the individual in order to obtain a reconstructed modulation signal;
calculating (415) a degree of statistical dependence between the reconstructed modulation signal and each modulation signal of the set of at least one modulation signal;
identifying (415) at least one visual stimulus corresponding to a modulation signal for which the degree of statistical dependence is greater than a first threshold.

2. Method according to claim 1, wherein the set of at least one visual stimulus comprises a plurality of visual stimuli and the set of at least one modulation signal comprises a plurality of modulation signals, the method comprising:

searching (415), among the plurality of modulation signals, for a modulation signal for which a degree of statistical dependence on the reconstructed modulation sigal is maximum; and
identifying (415) the visual stimulus corresponding to the modulation signal for which the degree of statistical dependence is maximum;
the modulation signals being composed such that an overall degree of statistical dependence, determined in the time and/or frequency domain, for all pairs of modulation signals corresponding to two distinct visual stimuli is less than a second threshold.

3. Method according to claim 1 or 2, wherein the reconstruction is carried out by applying a reconstruction model to the plurality of electroencephalographic signals.

4. Method according to claim 3, wherein the reconstruction model comprises a plurality of parameters for combining electroencephalographic signals, the method comprising determining values plurality of parameters for combining electroencephalographic signals during an initial learning phase.

5. Method according to claim 4, the method also comprising, during the initial learning phase applied to a subset of at least one visual stimulus among a plurality of visual stimuli,

obtaining, for each visual stimulus of said subset of at least one visual stimulus, electroencephalographic test signals produced by the indivudal focusing their attention on the visual stimulus in question; and
determining optimal values from the plurality of parameters for combining electroencephalographic signals for which the application of the reconstruction model to the plurality of electroencephalographic test signals recorded for a visual stimulus makes it possible to generate a reconstructed modulation signal that best approximates the modulation signal corresponding to the visual stimulus in question.

6. Method according to any of the preceding claims, wherein each modulation signal defines variations as a function of the time of at least one parameter for applying an elementary transformation to a graphical object.

7. Method according to claim 6, wherein an application parameter is a degree of transformation or a speed of application of an elementary transformation.

8. Method according to any of the preceding claims, wherein an elementary transformation is a transformation of the set of transformations that consists of varying the light intensity, varying the contrast, colorimetric transformation, geometric deformation, rotation, oscillation, displacement along a trajectory, a change of shape and a change of graphical object or a combination of transformations chosen from within said set of transformations.

9. Computer programme comprising programme code instructions for executing steps of a method according to any of the preceding claims when said computer programme is executed by a data processor.

10. Computer system comprising at least one memory for storing code instructions for a computer programme, said computer system being configured to execute a method according to any of claims 1 to 7, and at least one data processor that is configured to execute said computer programme.

11. System for determining the focus of an individual's visual attention based on electroencephalographic signals, the system comprising

a device (110) for generating display signals that is configured to generate (410) a set of at least one visual stimulus to be displayed from at least one graphical object, from at least one elementary transformation and from a set of at least one modulation signal, a visual stimulus being an animated graphical object obtained by applying to a graphical object a temporal sequence of elementary transformations that is temporally parameterised by a corresponding modulation signal;
a device (120) for proessing signals that is configured to

- obtain (411) a plurality of electroencephalographic signals produced by the individual;
- obtain (414) a reconstructed modulation signal by reconstructing a modulation signal based on the plurality of electroencephalographic signals;
- calculate (415) a degree of statistical dependence between the reconstructed modulation signal and each modulation signal of said set of at least one modulation signal; and
- identify (415) at least one visual stimulus corresponding to a modulation signal for which the degree of statistical dependence is greater than a first threshold.

12. System according to claim 11, wherein the set of at least one visual stimulus comprises a plurality of visual stimuli and the set of at least one modulation signal comprises a plurality of modulation signals, the device (120) for processing signals also being configured to

- search (415), among the plurality of modulation signals, for the modulation signal for which the degree of statistical dependence on the reconstructed modulation sigal is maximum;
- identify (415) the visual stimulus corresponding to the modulation signal for which the degree of statistical dependence is maximum;

the modulation signals being composed such that an overall degree of statistical dependence, determined in the time and/or frequency domain, for all pairs of modulation signals corresponding to two distinct visual stimuli is less than a second threshold.

EP 3 678 532 B1

105

110

Dispositif de génération
de signaux d'affichage

130

101

Dispositif de
commande

140

Dispositif de traitement
de signaux

120

FIG.1A

1 2 3
4 5 6
7 8 9
0

FIG.1B

EP 3 678 532 B1

O1, O2, ... ON

Objets graphiques

1 2 3
4 5 6
7 8 9

OA1, OA2, ... OAN

Objets graphiques animés

S1, S2, ... SN

Signaux d'affichage

105

Affichage

1 2 3
4 5 6
7 8 9
0

101

Transformation(s)

T1, T2, ... TP

SM1, SM2, ... SMN

Signaux de modulation

EEG

E1, E2, ... EX

Signal de modulation reconstruit

SMR

Modèle de recontruction

MR

Paramètres de modèle

P1, P2, ... PK

FIG.2A

FIG.2B

FIG.2C

FIG.2D

FIG.2E

FIG.3

Essai i: Enregistrement des données d'entraînement: segments EEG $E_{i,j}$ horodatés et signal de modulation correspondants $SM_i$ —401

Synchronisation entre les segments d'EEG $E_{i,j}$ et le signal de modulation correspondants $SM_i$ —402

Concaténation des segments d'EEG $E_{i,j}$ et des signaux de modulation correspondants —403

Prétraitement et débruitage des signaux EEG —404

Détermination de la combinaison optimale des signaux EEG pour reconstruire les signaux de modulation —405

Modèle mathématique entraîné et utilisable en temps réel

FIG.4A

Génération d'au moins un stimulus visuel à partir d'au moins un objet graphique et d'au moins un signal de modulation ⟍411

↓

Enregistrement de signaux EEG ⟍412

↓

Prétraitement et débruitage des signaux EEG ⟍413

↓

Reconstruction d'un signal de modulation à partir des signaux EEG ⟍414

↓

Recherche d'une corrélation entre le signal de modulation reconstruit et au moins un signal de modulation ⟍415

↓

Identification du stimulus visuel sur lequel l'individu porte son attention

# FIG.4B

APP 1 APP 2 APP 3 APP 4

APP 5 APP 6 APP 7 APP 8

APP 9 APP 10 APP 11 APP 12

APP 8 APP 8 APP 8 APP 8 APP 8

DÉROULÉ TEMPOREL

FIG.5

EP 3 678 532 B1

A: sans crowding

B: avec crowding

FIG.6

EP 3 678 532 B1

FIG.7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8391966 B2

**Littérature non-brevet citée dans la description**

- An online multi-channel SSVEP-based brain computer interface using canonical corrélation analysis method. **GUANGYU BIN et al.** Journal of Neural Engineering. IOP Publishing, 2009